(19) Europäisches Patentamt
European Patent Office
Office européen des brevets

(11) Publication number: **0 035 719**
**B1**

(12) **EUROPEAN PATENT SPECIFICATION**

(45) Date of publication of patent specification: **20.09.89**

(51) Int. Cl.$^4$: **C 07 H 21/00**, C 07 H 19/04, C 01 B 33/12, C 07 H 23/00

(21) Application number: **81101445.5**

(22) Date of filing: **27.02.81**

(60) **Divisional applications 85200673 filed on 01.05.85, 83104852 filed on 17.05.83.**

(54) **Process for producing modified inorganic polymers, their use in producing polynucleotides, and a reagent useful in these processes.**

(30) Priority: **29.02.80 US 126025**

(43) Date of publication of application:
**16.09.81 Bulletin 81/37**

(45) Publication of the grant of the patent:
**20.09.89 Bulletin 89/38**

(84) Designated Contracting States:
**AT BE CH DE FR GB IT LI NL**

(56) References cited:
**EP-A-0 040 099**
**EP-A-0 061 746**
**DE-B-1 113 937**

**Nucleic Acid Research, 7 (1979), 1955-64,**

**Tetrahedron Letters 21 (1980) 2265-8**

**The file contains technical information submitted after the application was filed and not included in this specification**

(73) Proprietor: **UNIVERSITY PATENTS, INC.**
**537 Newtown Avenue**
**Norwalk Connecticut 06851 (US)**

(72) Inventor: **Caruthers, Marvin H.**
**1450 Kendall Drive**
**Boulder Colorado (US)**
Inventor: **Matteucci, Mark D.**
**3490 Berkley**
**Boulder Colorado (US)**

(74) Representative: **Allam, Peter Clerk et al**
**LLOYD WISE, TREGEAR & CO. Norman House**
**105-109 Strand**
**London WC2R 0AE (GB)**

(56) References cited:
**TETRAHEDRON LETTERS, no. 16, 1972, pages 1527-1530, Pergamon Press, GB. H. KOSTER: "Polymer support oligonucleotide synthesis. VI 1-5). Use of inorganic carriers"**

**CHEMICAL REVIEWS, vol. 77, no. 2, April 1977, pages 183-217, V. AMARNATH et al.: "Chemical synthesis of oligonucleotides"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

(56) References cited:
ADVANCES IN CARBOHYDRATE CHEMISTRY AND BIOCHEMISTRY, vol. 36, 1979, Ed. R.S. TIPSON and D. HORTON, pages 135-213, Academic Press, M. IKEHARA et al.: "Synthesis of polynucleotides"

TETRAHEDRON LETTERS, no. 16, 1972, pages 1531-1534, Pergamon Press, GB. H. KOSTER et al.: "Polymer support oligonucleotide synthesis. VIII). Use of Sephadex LH 20"

JOURNAL OF THE AMERICAN CHEMICAL SOCIETY, vol. 101:22, October 24, 1979, pages 6606-6613. P. TUNDO et al.: "Synthesis catalytic activity, and behaviour of phase-transfer catalysts supported on silica gel. Strong influence of substrate adsorption on the polar polymeric matrix on the efficiency of the immobilized phosphonium salts"

CHEMICAL ABSTRACTS, vol. 87, 1977, page 180, abstract 2035k, COLUMBUS, OHIO, US JOURNAL OF THE AMERICAN CHEMICAL SOCIETY, vol. 98:12, June 9, 1976, pages 3655-3661. R.L. LETSINGER et al.: "Synthesis of thymidine oligonucleotides by phosphite triester intermediates"

TETRAHEDRON LETTERS, no. 5, 1978, Pergamon Press, GB. G.S. BAJWA et al.: "Thymidine nucleoside 3',5'-cyclic phosphoramidites and phosphites. Configuration at phosphorus in trivalent and pentavalent cyclic nucleotides by 31p and 13c nmr", pages 421-424

TETRAHEDRON LETTERS, vol. 21, 1980, pages 719-722, Pergamon Press, GB. M.D. MATTEUCCI et al.: "The synthesis of oligodeoxypyrimidines on a polymer support"

**Description**

The present invention relates to a process of making modified inorganic polymers, and to a process of producing polynucleotides utilizing said modified inorganic polymers as a support structure and to nucleoside phosphite compounds useful as reagents in these processes.

Numerous attempts have been made to develop a successful methodology for synthesizing sequence defined oligonucleotides. However, the stepwise synthesis of polynucleotides, and specifically oligonucleotides still remain a difficult and time consuming task, often with low yields. One prior art technique has included the use of organic polymers as supports during polynucleotide synthesis. Classically the major problem with polymer supported synthesis strategies has been inherent in the nature of the polymer support. Various prior art polymers used in such syntheses have proven inadequate for reasons such as: (1) slow diffusion rates of activated nucleotides into the support; (2) excessive swelling of various macroporous, low cross-linked support polymers; and (3) irreversible absorption of reagents onto the polymer. See for example, V. Amarnath and A. D. Broom, *Chemical Reviews 77*, 183—217 (1977).

Modified inorganic polymers are known in the prior art, primarily for use as absorption materials, for example, in liquid chromatography. The attachment of nucleosidephosphates to silica gel using a trityl linking group is described in the prior art (H. Koster, *Tetrahedron* Letters, 1527—1530, 1972) but the method is apparently applicable only to pyrimidine nucleosides. The cleavage of the nucleoside from the silica support can only be accomplished with acid to which the purine nucleosides are sensitive.

The production of phosphotriester derivatives of oligothymidylates is described in the literature (R. L. Letsinger and W. B. Lunsford, Journal of the American Chemical Society, 98:12, 3655—3661) by reaction of a phosphorodichloridite with a 5'-O blocked thymidine and subsequent reaction of the product with a 3'-O— blocked thymidine followed by oxidation of the resulting phosphite to a phosphate and removal of blocking groups to obtain the phosphotriesters; using this procedure, the tetramer and pentamer products, dTpTpTpT and dTpTpTpTpT in which T is thymidine were prepared. Unfortunately, the process requires separation and purification of products at each stage to ensure proper sequencing of the added nucleosides. Separation techniques including precipitation and washing of precipitates are necessary to implement each successive stage reaction.

Recently, Matteucci and Caruthers have disclosed a process for the synthesis of oligodeoxypyrimidines on a polymer support (Tetrahedron Letters, Vol. 21, pps. 719—722, 1980). This process is illustrated by the following reaction scheme:

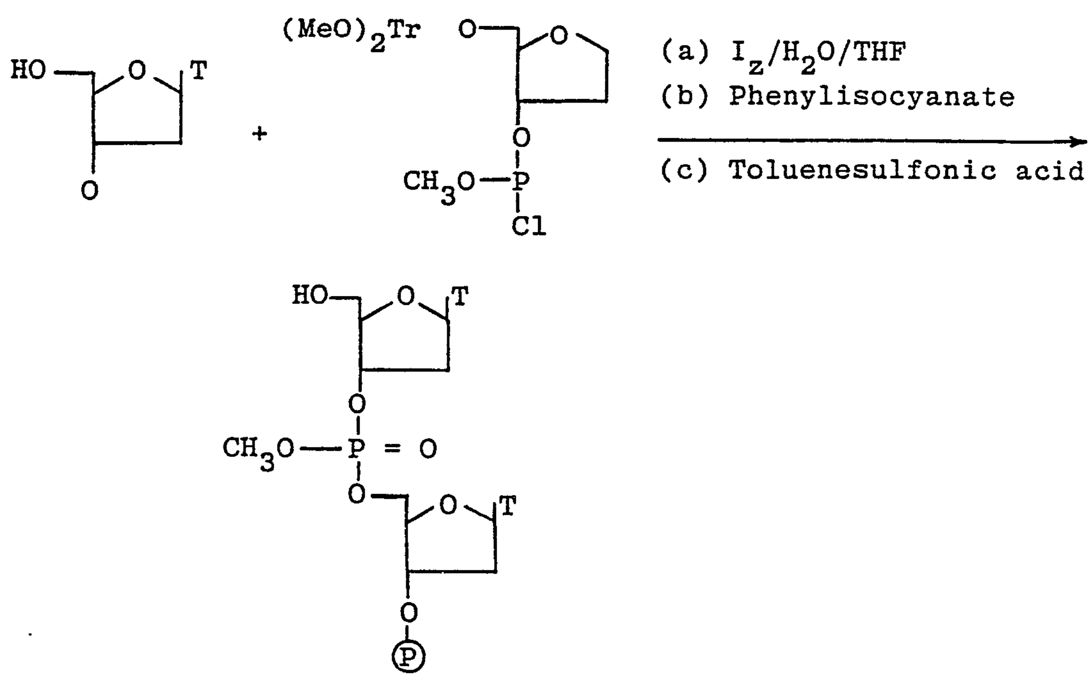


where Ⓟ represents silica gel and T represents thymidine. The product, a dinucleotide modified inorganic polymer, was then employed, using the same general procedure, to preparing a number of longer-chain oligonucleotides, for instance d(T-C-T-C-T-C-T-T-T).

Although the procedure of Matteucci and Caruthers described in the Tetrahedron Letters paper, *supra*, represented an advance in the art of oligonucleotide synthesis, it is found that the procedures employed cannot add guanosine units in acceptable yields in the synthesis of DNA.

Organic phosphoramidites are a known class of compounds, and their reactions with alcohols to form phosphite triesters has been reported by a number of Russian works in Zhurnal Obschei Khimii, for example in Vol. 39 (1969), pages 854—856, vol. 43 (1973), pages 55—59, Vol. 43 (1973), page 498, Vol. 45 (1975), pages 2338—2339 and Vol. 46 (1976), pages 2204—2207. English translations of these papers have been published by Plenum Publishing Corporation of New York, USA. However, prior to the present invention the use of this alcoholysis reaction in producing polynucleotides has not been known, and indeed was contraindicated by the work reported by Gough *et al* in Nucleic Acids Research Vol. 7, No. 7, (1979), at pages 1955—1964.

In accordance with one aspect, the present invention provides a process for preparing a modified inorganic polymer represented by the formula:

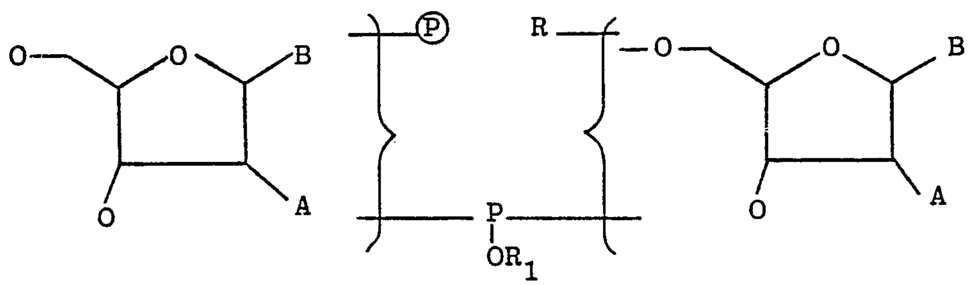

wherein Ⓟ is an inorganic polymer linked to the 3'- or 5'-O of the nucleoside through a hydrolyzable covalent bond; R is H or a blocking group; R is a hydrocarbyl radical containing up to 10 carbon atoms; each B is a nucleoside or deoxynucleoside base; and each A is H or OR; which process comprises condensing a compound of the formula:

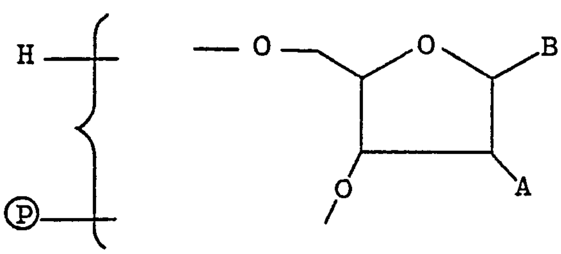

with a compound of the formula (I):

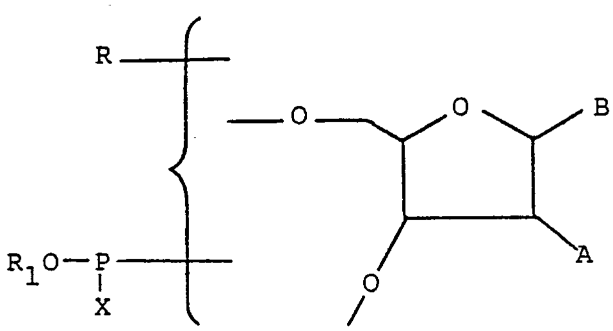

(I)

wherein A, B, Ⓟ, R and $R_1$ are as previously defined, and X is a secondary amino group attached through the amino nitrogen.

The invention further provides a process for the production of an oligonucleotide or polynucleotide, which comprises the steps of:

(1) condensing the 3'-OH or 5'-OH of a nucleoside or oligonucleotide covalently linked to an inorganic polymer by a coupling agent through the 5'-O— or 3'-O—, respectively, of said nucleoside or oligonucleotide with a phosphite compound of the formula (I):

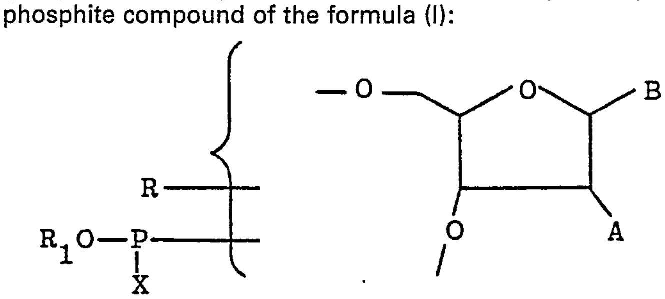

(I)

wherein R is a blocking group; B is a nucleoside or deoxynucleoside base; A is H or OR; $R_1$ is a hydrocarbyl radical containing up to 10 carbon atoms; and X is a secondary amino group; to form a product of the formula (II)

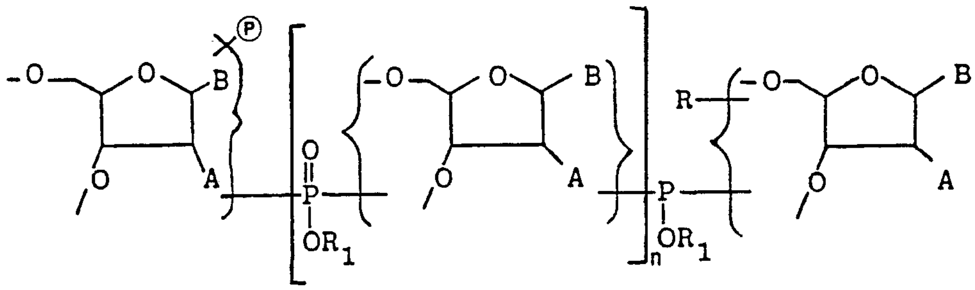

(II)

wherein ℗ represents the inorganic polymer, and n is an integer of at least one;

(2) oxidizing the terminal phosphite linkage of the product of step (1) to a phosphate linkage;

(3) removing the 3′-O— or 5′-O— blocking group of the product thus obtained;

(4) condensing the resulting product with a further nucleoside or oligonucleoside phosphite of formula (I), to form a product of formula (II) wherein n is an integer one greater than in the product of step (1);

(5) oxidizing the resulting phosphite triester to a phosphite triester;

(6) removing the 3′-O or 5′-O blocking group of the product thus obtained; and

(7) if desired repeating each of steps (4), (5) and (6) successively to add a desired sequence of nucleosides to the chain; with the proviso that step (6) may be omitted following the addition of the final nucleoside to the chain.

In a product aspect, the present invention provides a compound of formula (I):

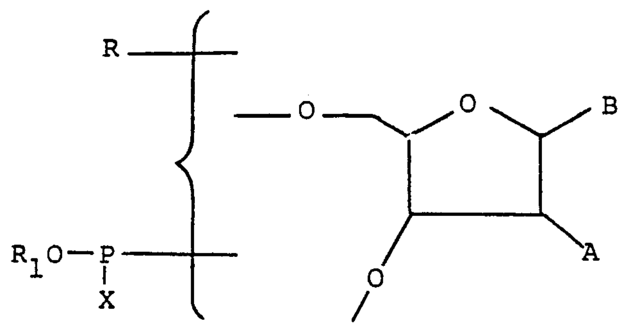

(I)

wherein B, R, A, $R_1$ and X are as defined above. As indicated above, compounds of formula (I) are useful reagents in the preparation both of modified inorganic polymers and of oligonucleotides or polynucleotides by the process of this invention.

In general, the modified inorganic polymer supports with which the invention in certain aspects is concerned comprise the inorganic polymer to which is chemically bound a nucleoside. The chemical bonding of the nucleoside moiety to the polymer is by means of reactive groups on the polymer which react with reactive groups of the nucleoside molecule. Representative combinations of such groups are amino with carboxy to form amide linkages between the nucleoside and the support, or hydroxy with carboxy to form ester linkages between the respective moieties.

To accomplish the requisite chemical bonding, each of the reactants must of course contain the necessary reactive groups. Thus, the polymer support can be provided with terminal carboxy functionality which will react with hydroxy and/or amino groups of the nucleoside. Alternatively, the nucleoside can be provided with carboxy functionality by acylation of the hydroxyl and/or amino groups using a dicarboxylic acid and the carboxy-functional nucleoside reacted with hydroxy or amino groups of the polymer support. Hydroxy and amino functionality where not present on the inorganic support can be introduced by known methods. For example, with silica supports, amino functionality can be introduced by reaction with aminoalkylsilyl halides.

Of course, the nucleoside moiety of the present modified inorganic polymers can include more than one nucleoside and may include a number of nucleosides condensed as oligonucleotides with the oligonucleotide being attached to the inorganic polymer support through the single chemical linkage, e.g. ester linkage.

The thus modified inorganic polymer supports are useful in the stepwise addition of nucleosides or oligonucleotides to the original nucleoside moiety of the support by a series of process steps as described hereinafter. Subsequently, the polynucleotides so produced are released from the polymer support and recovered from the polymer by a series of process steps including alkali hydrolysis of the chemical bond between the polynucleotide and the support.

The present invention is particularly useful in the chemical synthesis of any deoxyribonucleic acids (DNA) and ribonucleic acids (RNA) which contain any deoxynucleotides, nucleotide, polynucleotide, and polydeoxynucleotide. Thus natural DNA and RNA as well as new DNA and RNA can be synthesized.

A wide variety of inorganic polymers can be employed in the present invention and these include, for example, silica, porous glass, aluminosilicates, borosilicates, metal oxides such as alumina and nickel oxide, and various clays. The polymer should be substantially insoluble in the reaction solvents employed and relatively chemically inert to the reagents employed during processing, except of course for the chemical reactivity required to form the hereinbefore-described chemical bond with the initial nucleoside through which the eventual polynucleoside is attached to the support.

The process for preparing oligonucleotides or polynucleotides of the present invention is accomplished by treatment of the nucleotide or nucleoside-modified inorganic polymer support by a series of sequential steps whereby each series results in the addition of nucleotide to the modified support until the desired sequence of nucleotides is obtained. The series of sequential steps is as follows:

(a) coupling of a selected nucleoside through a phosphite linkage to the nucleoside bound to the polymer support, i.e. the nucleoside-modified support;

(b) optionally, but preferably blocking unreacted hydroxyl groups on the nucleotide of the polymer support;

(c) oxidation of the phosphite linkage of step (a) to form a phosphate linkage.

(d) removal of protecting group from the selected nucleoside described in step (a) to regenerate a reactive site for the next cycle of these steps.

Each nucleoside is added sequentially to the polymer support by repetition of steps a, b, c and d until the final oligonucleotide is obtained, after which the oligonucleotide may then be removed from the support by hydrolysis reaction which can also remove blocking groups from the oligonucleotide molecule. The removal of blocking groups and hydrolytic cleavage of the oligonucleotide from the support can be accomplished stepwise, which is preferred, or in a single hydrolysis reaction.

The nucleoside-modified support is prepared by covalently coupling a nucleoside to the inorganic polymer using a suitable coupling agent through the 3'- or the 5'-OH of the starting nucleoside. This is accomplished with the starting nucleoside being blocked in either the 3'- or the 5'-OH, and the nucleoside is coupled through the unblocked hydroxy group to the polymer support by the coupling agent. After condensation, residual reactive groups, e.g. carboxy groups, which did not react can be blocked by suitable means, e.g. conversion of carboxy groups to carboxyamide by reaction with simple amines. Thereafter, the blocked 3'- or 5'-hydroxy group is converted to free hydroxy by removal of the blocking group and the free hydroxy group is available for condensation with a selected nucleoside containing a phosphite linking groups as in step (a) hereinbefore described.

A variety of coupling agents or groups on the polymer support can be used to covalently link the initial nucleoside or oligonucleoside to the polymer support. Representative groups include amino, especially primary amino, hydroxy, thiol, sulfonic acid, phosphorous and phosphoric acid, particularly in the form of acid halides, especially chloro and bromo and carboxy, among others. These reactive groups are conveniently attached to the polymer support commonly through a hydrocarbyl radical such as an alkylene or phenylene divalent radical, one valence position being occupied by the chain bonding and the remaining attached to the reactive groups. Such hydrocarbyl groups may contain up to about 10 carbon atoms, preferably up to about 6 carbon atoms. Alkylene radicals are usually preferred containing 2—4 carbon atoms in the principal chain.

The nature of the reactive group which bonds the nucleoside to the polymer support is not critical provided that it is readily hydrolyzable to permit separation of the oligonucleotide product from the polymer support at the conclusion of the preparative process.

If desired, the aforesaid coupling groups can be present on the nucleoside for reaction with reactive groups, e.g. hydroxy or amino, on the support polymer. Normally it is preferred to have the coupling groups on the polymer support.

The process of this invention is particularly advantageous in that it provides a rapid synthetic route to oligonucleotides and oligodeoxynucleotides which is characterized by high yields and high purity. Each mononucleotide addition requires maximally 2—3 hours with yields of 95% and greater being obtained for each addition. Further, these same yields are obtained as the oligonucleotide grows in size.

While the invention can be implemented with a variety of inorganic polymers, it will be described herein in more detail utilizing silica gel as the polymer support. A particularly preferred silica gel is macroporous silica which is used in high performance liquid chromatography (hplc). In addition, the invention will be described using deoxynucleotides but it should be understood that ribonucleotides can be substituted therefor to obtain similar results.

As employed herein, the terms nucleoside, nucleotide and oligonucleotide are intended to include the deoxy counterparts which differ only in the absence of a hydroxy group in the 2' position. Thus, these terms include structures wherein the 2' position substituent is H or OH (as shown hereinafter by substituent A in formulae I, II and III).

(A) Preparation of nucleoside-modified support.

The silica gel support is linked to the nucleoside through a linkage which is readily hydrolyzable,

preferably with a weak base such as ammonium hydroxide. The most preferred linkage is an ester linkage which readily hydrolyzes in a weak base such as ammonium hydroxide. This linkage can be accomplished by first linking carboxy functionality to the support or by preforming the ester linkage on the nucleoside by esterification followed by condensation of the esterified nucleoside through the esterifying acid moiety to the support.

The first of these embodiments can be accomplished by the following steps:

(1) conversion of silica gel to a matrix containing aminoalkyl groups or hydroxyalkyl groups covalently bound thereto;

(2) reaction of the aminoalkyl silica with a dicarboxylic acid to form an amide or ester linkage and carboxy functionality;

(3) blocking unreacted silanol OH groups;

(4) condensation of the free carboxy groups of the silica with the free hydroxy (3'- or 5'-) of the selected nucleoside; and

(5) blocking unreacted carboxy groups by conversion to unreactive derivatives, e.g. amides. The alternative embodiment involves the following steps:

(1) conversion of silica gel to matrix containing aminoalkyl groups or hydroxyalkyl groups;

(2) block unreacted silanol OH groups;

(3) join the derivatized silica gel through amide or ester formation to the free carboxy group of a selected nucleoside which has been modified to contain the half ester of a dicarboxylic acid; and

(4) blocking unreactive amino or hydroxy groups on the silica gel support, e.g. using acetic anhydride. Both embodiments give the same product from identical reactants. The second embodiment however is preferred since it leads to more control of the amount of nucleoside loaded onto the silica gel. Additionally, the second embodiment leads to more nucleoside joined to the silica gel (approximately 100—120 μmole/g compared to 10—40 μmole/g by the first embodiment).

Preferably, the nucleoside is linked to the silica gel through the 3'-OH group rather than the 5'-OH leaving the 5'-OH available for linkage through phosphite to the added nucleoside. Thus, linkage of the added nucleoside occurs at the 3'-OH group and the 5'-OH remains available for linkage to a further added nucleoside.

Accordingly, to accomplish the desired linkages at the 3'-OH and 5'-OH respectively, the initial nucleoside is linked through the 3'-OH to the silica gel by the coupling reaction previously defined herein. This is accomplished by blocking the 5'-OH e.g. by use of trityl groups, such as the dimethoxytrityl group, which are preferred since they are readily removed after the initial 3'-OH coupling reaction occurs.

When the initial nucleoside includes amino groups, e.g. guanosine, adenosine, cytidine, deoxyguanosine, deoxyadenosine and deoxycytidine, it is preferred to block these groups using known acylating techniques, e.g. with acetic aacid, benzoic acid, isobutyric acid and like acids and such blocking group can be removed when convenient, usually after the final oligonucleotide is obtained.

The aminoalkyl groups are incorporated on the silica gel by reaction of aminoalkyl-trialkoxysilane which is conveniently accomplished by refluxing in a solvent, e.g. toluene, for several hours. Suitable reagents include aminopropyltriethoxysilane, 4-aminobutyltrimethoxysilane, 4-aminobutyltriethoxysilane, 2-aminoethyltriethoxysilane and others.

The dicarboxylic acid employed in forming the ester linkage of deoxynucleoside to the silica gel can be any of a variety such as succinic, glutaric, adipic, phthalic, maleic and similar such dicarboxylic acids of the aliphatic or aromatic type containing preferably up to about 10 carbon atoms. Esterification with the dicarboxylic acid is best accomplished by using the acid anhydride to assure monoesterification.

The product produced, i.e. the nucleoside-modified silica gel, can be represented by the following formula:

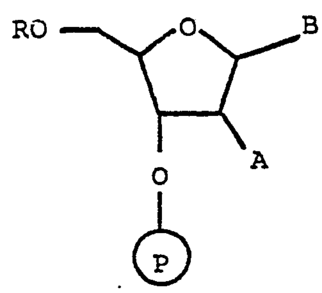

wherein B is the nucleoside or deoxynucleoside base; Ⓟ represents the silica support and the covalent linking group which is preferably represented by the formula

$$(\text{silica gel})\text{——}(CH_2)_n NHCO\ Z\ CO\text{—}$$

in which n is an integer from 1—5 and Z is divalent hydrocarbyl radical including alkyl, alkenyl, cycloalkyl, aryl and aralkyl of up to about 10 carbon atoms; A is H or OR; and R is H or a blocking group, e.g. trityl, methoxytrityl, dimethoxytrityl, dialkylphosphite, pivalylisobutyloxycarbonyl, t-butyldimethylsilyl, and similar such blocking groups.

Formation of phosphitic-linked oligonucleosides

The deoxynucleoside-modified silica gel is condensed with a selected nucleoside through formation of a triester phosphite linkage between the 5′-OH of the deoxynucleoside of the silica gel and the 3′-OH of the selected deoxynucleoside. The phosphite linkage can be produced by first incorporating the phosphite group onto the 5′-OH of the nucleoside on the silica gel followed by condensation with the added nucleoside through the 3′-OH. Alternatively, and preferably, the phosphite group is incorporated into the added nucleoside at the 3′-OH (the 5′-OH being blocked as by tritylating) and the resulting nucleoside phosphite then reacted with the 5′-OH of the nucleoside on the silica gel.

The deoxynucleoside-modified silica gel can also be condensed with a selected nucleoside through formation of a triester phosphite linkage between the 3′-OH of the deoxynucleoside of the silica gel and the 5′-OH of the selected deoxynucleoside. The phosphite linkage can be produced by first incorporating the phosphite group onto the 3′-OH of the nucleoside on the silica gel followed by condensation with the added nucleoside through the 5′-OH. Alternatively and preferably by this approach, the phosphite group is incorporated into the added nucleoside at the 5′-OH (the 3′-OH being blocked as by tritylating using art from procedures) and the resulting nucleoside phosphite then reacted with the 3′-OH of the nucleoside on the silica gel.

The general reaction can be represented by the following

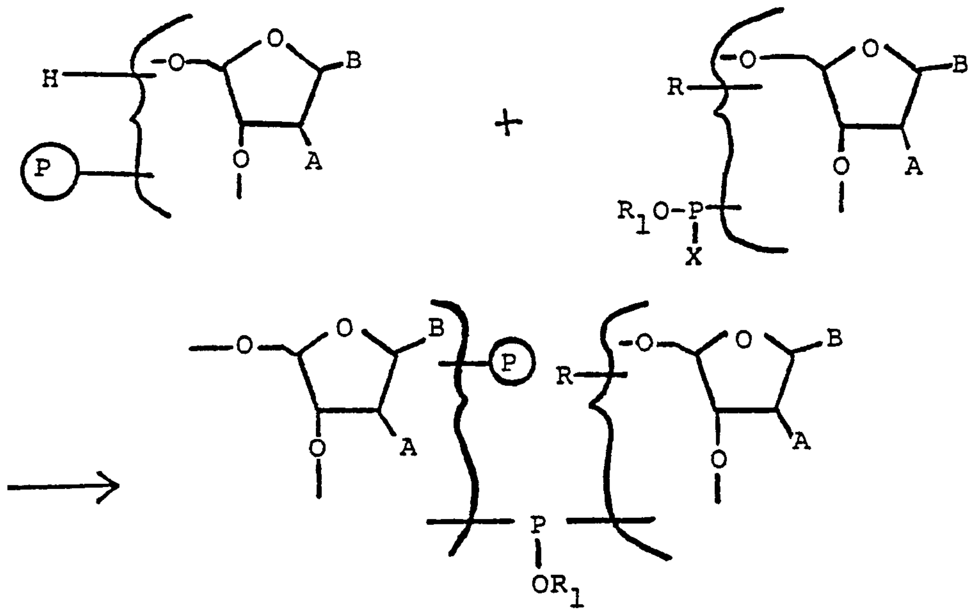

The preferred reaction is represented as follows:

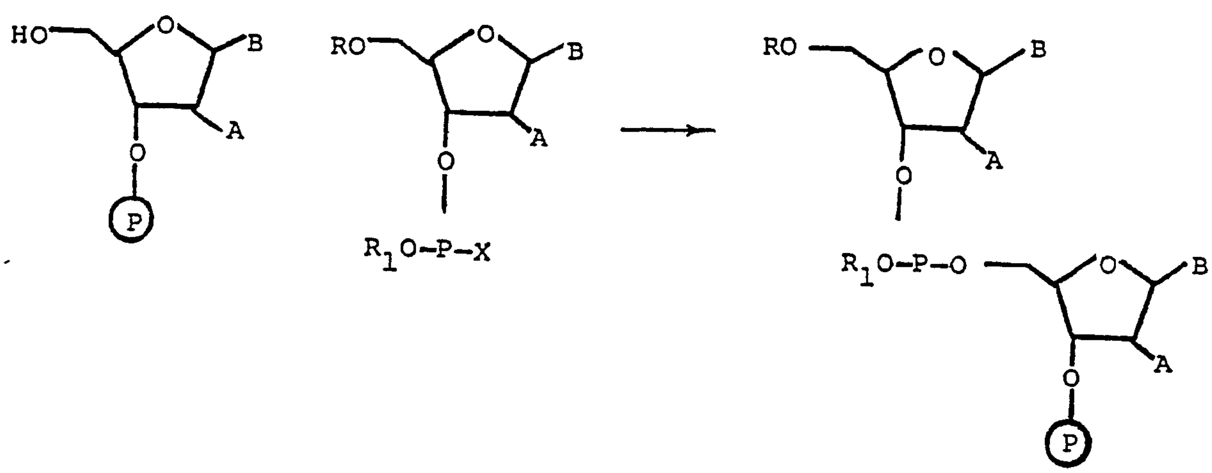

wherein A, B and Ⓟ are as previously defined, R is a blocking group as previously defined, $R_1$ is a hydrocarbyl radical containing up to 10 carbon atoms, preferably lower alkyl and X is a secondary amino group attached through the amino nitrogen. The secondary amino group represented by substituent X is preferably one which is formed by removal of the hydrogen atom from a ring nitrogen of a nitrogen heterocyclic compound which contains unsaturated bonds in the ring structure. Exemplary nitrogen-containing heterocyclics include tetrazole, substituted imidazoles such as nitroimidazole, indole pyrazole, imidazole, benzimidazole, isoindole, pyrrole, triazole, dioxazole and similar heterocyclics, as well as analogs and homologs thereof.

When X is such a secondary group, the resulting product is very reactive and somewhat unstable at ordinary temperatures. In present experience, these compounds should be freshly prepared as needed, or alternatively they can be prepared, isolated and stored in sealed containers at reduced temperature, usually well below 0°C and usually at about −20°C.

The removal of the blocking group R permits reaction with a further nucleoside i.e. a compound of formula I above and repeat reaction gives rise to the polynucleotide of determined sequence of nucleotides attached to the silica gel through the covalently-bonded linking group, e.g. ester linking group.

The phosphite linking group is introduced into the nucleoside moiety of the silica gel at the 5'-OH position or the 3'-OH position of the added nucleoside by reaction with a hydrocarbyl (as previously defined herein) phosphorodichloridite, e.g. methyl phosphorodichloridite, preferably in the presence of a base, such as an organic amine. The resulting nucleoside compound of formula I can be stored in solvent for about one week under an inert gas such as nitrogen or argon and temperatures below about −20°C.

The reaction of the deoxynucleoside-modified polymer with the nucleus phosphite compound of formula I is effected in the presence of a base, such as an organic amine, preferably tertiary organic amines, e.g. pyridine, lutidines and similar amines.

Blocking Reactions

After condensation of the selected nucleoside through phosphite linkage to the nucleoside or oligonucleotide attached to the silica gel support, a small but significant amount (about 1—5%) of the nucleoside or oligonucleotide attached to the silica gel does not react with the added nucleoside. These unreactive moieties preferably are capped or blocked in order to prevent the formation of several deoxyoligonucleotides with heterogeneous sequences. This capping or blocking step can be accomplished by reaction with a very reactive phosphite to form a 5'-phosphite ester group, a relatively nonhydrophobic triester. For example, diethoxytriazolylphosphine can be used to form the diethyl phosphite-5'-deoxynucleoside triester. Corresponding di-lower alkoxy nitrogen-containing heterocyclylphosphines can be used in lieu of the triazolyl phosphine, e.g. tetrazolyl, imidazolyl and 4-nitroimidazolyl phosphine, to produce the corresponding di-lower alkyl triester. These nitrogen-heterocycl phosphines are prepared from the corresponding phosphinyl chloride, of course, the phosphinyl chloride can be used to phosphinylate the nucleoside but the nitrogen heterocyclyl phosphines are preferred since their use leads to higher yield.

More traditional blocking or capping groups can be employed such as acid anhydrides like acetic anhydride and arylisocyanates like phenyl isocyanate but these react more slowly with the unblocked 5'-hydroxy group. When acetylation with acid anhydrides, e.g. acetic anhydride, is conducted in the presence of tertiary amines, especially di-loweralkylaminopyridines like dimethylaminopyridine, acylation occurs rapidly and this procedure is preferred for blocking especially the 5'-hydroxy group. The dialkylphosphite capping group can also be used. The resulting triester is relatively nonhydrophobic and a preferred purification involves reverse phase high performance liquid chromatography which assures separation of the nonhydrophobic by-product from the product containing the hydrophobic 5'-O-dimethoxytrityl group.

To block unreacted silanol hydroxy groups on the silica gel before nucleoside addition, the use of trialkoxysilyl chloride is preferred, although blocking can also be accomplished by acylation with hydrocarbylmonocarboxylic acids, preferably containing up to 10 carbon atoms, such as acetic, benzoic, butyric, isobutyric and naphthoic acids.

Oxidation of phosphite to phosphate

The oxidation is normally carried out using iodine as oxidizing agent using standard procedures. Alternatively, the oxidation can also be accomplished by reaction with peroxides like tertiary butyl peroxide and benzoyl peroxide as well as hydroperoxides. The use of hydrogen peroxide can lead to the formation of side products and is not preferred.

Oxidation should be effected before further condensation of nucleoside is attempted to obtain best yields. Attempts to defer oxidation until after all condensation reactions are completed have resulted in reduced yield of oligonucleotides due to formation of side products.

The removal of blocking groups is accomplished by art recognized procedures using mild bases such as ammonium hydroxide whether at room temperature or at elevated temperature.

In stepwise removal of blocking groups, it is preferred to first remove the alkyl group, e.g. methyl, from the phosphotriesters using triethylammonium thiophenoxide in solvent, e.g. dioxane or tetrahydrofuran. Thereafter, the product is treated with ammonium hydroxide at room temperature (20°C.) to hydrolyze the ester linkage joining the oligonucleotide to the support. Then N-acyl blocking groups, e.g. acetyl, benzoyl, and isobutyrl, are removed by warming at 50°C. for about 12 hours.

The removal of trityl blocking groups is conveniently effected employing Lewis acids, particularly zinc bromide, although other Lewis acids have also been effective, e.g. $AlCl_3$, $BF_3$ and $TiCl_4$. Usually nitromethane is used as solvent for this reaction although other solvents such as tetrahydrofuran can be used, as well as mixed solvents such as nitromethane and a lower alkanol, such as methanol. Alternatively, protic acids such as toluene-sulfonic acid can be used to remove the blocking group. With purine

nucleoside-containing products, however, some depurination can occur when protic acids are employed and therefore the use of Lewis acids is preferred for removal of the blocking group from purine containing products.

Employing the hereindescribed process, oligonucleotides containing up to 10—30 nucleoside units can be produced. The oligonucleotides can be converted by $T_4$-ligase and T-4 kinase to form a DNA sequence of choice by known enzymological reactions.

The products as obtained after hydrolysis can be purified by standard procedures after separation from the inorganic polymer support. The final purification is preferably by reverse phase hplc of the 5'-O-dimethoxytrityloligonucleotide as previously mentioned herein, followed by removal of the dimethoxytrityl group, e.g. using a lower alkanoic acid such as acetic acid.

The accompanying drawing is a schematic flow diagram of an apparatus which can be used to prepare oligonucleotides and polynucleotides by the process of the present invention.

More specifically, and with reference now to the drawing, an apparatus is illustrated to accomplish the foregoing. The column 10 is packed appropriately with solid silica gel matrix 12, derivatized as described herein.

Valve 14 is appropriately programmed under control of valve controller 15 to select among the four active reagents contained in reservoirs 16, 18, 20, and 22, and the wash solvents contained in reservoirs 24 and 26. Valve 14 will permit the independent selection from any reservoir in any order without the need to sequence through intervening reservoirs. Thus, for example, the reagent from reservoir 16 may be selected, and immediately thereafter the wash solvent from reservoir 24. These reagents are required for chain elongation in accordance with the teaching of the method of this invention and are maintained at room temperature for use therein.

Valve 28, is appropriately programmed under control of controller 15' to select among the five nucleoside-active phosphite triester contained in reservoirs 30, 32, 34, 36 and 38, and the wash solvent in reservoir 40. Once again, valve 28 permits independent selection (to prevent cross contamination) as described above. In addition, the reservoirs 30—38 are designed to maintain the adducts at −78°C. and the valve 28 to allow for the passage therethrough at this temperature.

Valve 42 is under the control of programmed controller 15'' for the selection of cleavage reagents contained in reservoirs 44 and 46 and a wash solvent in reservoir 48. These reagents and solvent are necessary to cleave the oligonucleotide from the support matrix 12 in column 10 and are maintained at room temperature.

The valve 50 operates in conjunction with pump 56 to selectively convey solvents, reagents or adducts from the valve 14, 28 and 42 towards the valve 52. In turn, this valve 52, under suitable control of a valve controller (not illustrated) selects between flow through the column 10 and uv detector 58 or recycling through column 10. Valve 54 is controlled to direct the flow from uv detector either to waste or collection. The uv detector 58 is utilized in the control of the system, through suitable feedback control means (not illustrated).

The programmed operation of the system is illustrated schematically in Table I below. This program illustrates the protocol for the addition of one nucleoside and then cleavage of the chain from support 12. It will be apparent that the system may be expanded and/or modified to accommodate the addition of more than one nucleotide, and that the entire system will preferably operate under control of a suitably programmed microprocessor computer.

The apparatus has particular applicability for automated operation, details of which are within the preview of one skilled in the art.

TABLE I

| Step | Duration | Valve 14, 28, 42/ Selection Anode | Valve 50 Selection Anode | Valve 52 Selection Anode | Valve 54 Selection Anode | Pump Speed |
|---|---|---|---|---|---|---|
| 1 | 2 min. | V-14/16 | V-14 | Flush | Waste | L |
| 2 | 60 min. | V-14/16 | V-14 | Recycle | " | L |
| 3 | 5 min. | V-14/24 | V-14 | F | " | H |
| 4 | 5 min. | V-14/26 | V-14 | F | " | H |
| 5 | 2 min. | V-14/18 | V-14 | F | " | L |
| 6 | 2 min. | V-14/18 | V-14 | R | " | L |
| 7 | 5 min. | V-14/26 | V-14 | F | " | H |
| 8 | 2 min. | V-14/20 | V-14 | F | " | L |
| 9 | 2 min. | V-14/20 | V-14 | R | " | L |
| 10 | 2 min. | V-14/24 | V-14 | F | " | H |
| 11 | 3 min. | V-28/40 | V-28 | F | " | H |
| 12 | 20 sec. | V-28/30 | V-28 | F | " | L |
| 13 | 5 sec. | V-28/30 | V-28 | F | " | L |
| 14 | 60 min. | V-28/30 | V-28 | F | " | L |
| 15 | 10 sec. | V-28/30 | V-28 | R | " | L |
| 16 | 30 sec. | V-28/30 | V-28 | F | Collect | L |
| 17 | Stop | | | | | |
| 18 | 2 min. | V-14/22 | V-14 | F | Waste | L |
| 19 | 30 min. | V-14/22 | V-14 | R | " | L |
| 20 | 5 min. | V-14/26 | V-14 | F | " | H |
| 21 | 5 min. | V-14/24 | V-14 | F | " | H |
| One Nucleotide Added; Cleavage Sequence: | | | | | | |
| 1 | 2 min. | V-42/44 | V-42 | F | Waste | L |
| 2 | 60 min. | V-42/44 | V-42 | R | " | L |
| 3 | 5 min. | V-42/48 | V-42 | F | " | H |
| 4 | 30 sec. | V-42/46 | V-42 | F | " | H |
| 5 | 180 min. | V-42/46 | V-42 | R | " | L |
| 6 | 10 min. | V-42/46 | V-42 | F | Collect | L |
| 7 | 10 min. | V-42/48 | V-42 | F | " | L |

The silica gel which is used as starting material in the production of the modified silica gels of the present invention is not critical. Silica gel particles in the range of from about 5 µm to about 1000 µm are useful, with particles in the range of about 10 µm to about 50 µm being preferred. In a similar manner pore size is not critical. Pore sizes in the range of about 50 Å to about 2000 Å are preferred.

The modified silica gels of the present invention: (1) allow relatively rapid diffusion of activated nucleotides, and other reagents into the support; (2) avoid swelling; and (3) resist adsorption of reagents. Additionally, the modified silica gels of the present invention are (1) insoluble in most solvents; (2) in use as support matrices, allow solvents and unwanted reaction products to be easily washed from the matrix, while maintaining the desired reaction products in place and capable of continuous processing; and (3) allow the supported material to react relatively rapidly and in high yield, for example, in cylindrical condensation.

The modified silica gel employed to react with the initial nucleoside of the oligonucleotide chain to form the initial reactive material is prepared by art-recognized procedures. The production of a variety of functional groups on the surface of the silica gel suitable for reaction with the hydroxy (3'- or 5'-) of the initial nucleoside can be effected using known methods, e.g. methods described in U.S. Patents 3,519,538; 3,419,517; 3,652,761; and 3,669,841.

The preferred method for the present invention is to introduce amino functionality on the silica by reaction with an aminoalkyl silane derivative, e.g. by reaction of a trialkoxy 3-aminopropylsilane such as triethoxy 3-amino-propylsilane with the silica support to form the covalent linkage:

$$\begin{array}{c} \quad\;\; OEt \\ \quad\;\; | \\ Si-O-Si-(CH_2)_3NH_2 \\ \quad\;\; | \\ \quad\;\; OEt \end{array}$$

and the amino group is reacted with one carboxy group of a dicarboxylic acid therefor giving rise to carboxy functionality on the silica gel where condensation of amino and carboxy occur. The silica gel can next be treated to block unreacted silanol groups with suitable blocking agents, e.g. trialkylhalosilanes such as trimethylchlorosilane or the bromo analog.

The resulting carboxy-derivatized silica can then be reacted with the hydroxy group (3'- or 5'-) of the first added nucleoside.

Alternatively, as previously indicated herein, the dicarboxylic acid can be reacted with the selected nucleoside to form a monoester at the 3'-O or 5'-O and the resulting ester containing a free carboxy group in the esterifying radical can be condensed with the amino-derivatized silica to form the same covalent linkage between nucleoside and silica support. Any unreacted amino groups of the amino-derivatized silica gel are preferably blocked by acylation with monocarboxylic acids such as acetic, benzoic or isobutyric acids normally employing the acid anhydrides under acylating conditions.

The structure of the covalent linkage between the first nucleoside and the silica support is not critical as long as a substantially stable but hydrolysable covalent linkage is formed to bind the nucleoside during the sequential nucleoside addition cycles. The covalent linkage therefore should be stable to the sequential reaction conditions but should be reasonably readily hydrolyzable to permit recovery of the formed oligonucleotide after completion of nucleoside addition. Thus, ester and amide linkages are particularly effective with the desired degree of stability and, at the same time, being readily hydrolyzable after nucleoside addition is completed using weak or strong bases.

As used herein the symbols for nucleotides and polynucleotides are according to the IUPAC—IUB Commission of Biochemical Nomenclature Recommendations [(1970) *Biochemistry 9,* 4022].

The following examples further illustrate the invention.

Example 1

A. Polymer supports functionalized with carboxylic acid groups are prepared from silica gel.

A separation group silica gel supplied by Vydak as TP silica, having 20 µm particle size, 300 Å pore size is used as the starting material. The silica gel was placed in a desicator over a saturated LiCl solution for 24 hours. The initial step in the process is silylation by refluxing 3-aminopropyltriethoxysilane (2.3 g, 0.01 M) with the silica gel (2.6 g) in dry toluene. After silylation is substantially complete, in this case after about twelve hours of refluxing, the reaction mixture is cooled and the toluene solution removed. The thus silylated silica gel is then washed serially with toluene, ethanol and then with ether and air dried. Succinic anhydride (2.5 g, 0.025 M) in water is next reacted with the silane modified silica gel to provide carboxylic acid functionality to the terminal portion of the covalently bonded silane side chains. During this latter reaction, the pH is maintained between 4 and 6 by addition of a base, such as 2 N sodium hydroxide. After this latter reaction, which proceeded for about 6 hours, the modified silica gel containing carboxylic acid functional groups on its side chains is washed with water, then with methanol and ether, and then finally dried in vacuum at room temperature. The modified silica gel is then treated with trimethylsilylchloride [$(CH_3)_3SiCl$, 1.09 g, 0.01 M] in anhydrous pyridine by refluxing for about 12 hours. The resulting modified

silica gel is then washed with 5% trichloroacetic acid in water, then with water, and then with ethanol and ether. After drying in vacuum, the yield of carboxylic acid functionality on the modified silica gel is about 250 μmole/g.

B. 5'-O-dimethoxytrityldeoxythymidine (1.17 g, 0.002 M) and the modified silica gel described in A (4 g, 0.001 mole carboxylic acid functional group) are reacted for about 40 hours in anhydrous pyridine using dicyclohexycarbodiimide (2.06 g, 0.01 M) as condensing agent. The unreacted residual carboxylic acid groups in the modified silica are blocked, by the addition of p-nitrophenol (1.4 g, 0.01 M) followed by the addition of 10% piperidine in pyridine (25 minutes). The reaction product is then washed serially with tetrahydrofuran, methanol and finally with ethyl ether. Then, as a precaution to assure complete blockage of unreacted carboxylic acid, the composition is first treated with dicyclohexylcarbodiimide and p-nitrophenol and then piperidine in pyridine for a second time. After removal of the dimethoxytrityl group using 0.1 N p-toluenesulfonic acid in acetonitrile, the yield of thymidine attached to the support is found by spectrophotometry to be about 40 μmole/g.

Example 2

A. Silica gel (Vydac A, 25 gms) was placed in a desicator over a saturated LiCl solution for 24 hr. The silica gel was transferred to a 500 ml round bottom flask, toluene (250 ml) and aminopropyltriethoxysilane (13 ml) were added, the flask was tightly sealed, and the suspension was gently shaken for 12 h at room temperature. The flask containing the suspended silica gel was next refluxed for 18 h. Before starting the reflux, add one boiling chip to the solution. Following the reflux step, the silica gel suspension was transferred to a centrifuge bottle and the silica gel pelleted by a low speed spin. The supernatant was decanted and the silica gel was washed with toluene (3 X, 80 ml ea), methanol (3 X, 80 ml ea) and methanol:$H_2O$, 1:1 (2 X, 80 ml ea). The silica gel was next suspended in 80 ml 50% aqueous methanol and shaken overnight at room temperature. Once again the silica gel suspension was isolated by transfer to a centrifuge bottle followed by a low speed spin. The silica gel was next washed with methanl (2 X, 80 ml ea) and ethyl ether (3 X, 80 ml ea). Finally, the silica gel was air dried for 6 h and then dried *in vacuo.*

The silica gel was placed in a round bottom flask. A solution of dry pyridine (50 ml) and trimethylsilyl chloride was added and the suspension shaken at room temperature overnight. The silica was isolated by low speed centrifugation. The silica was then washed with methanol (5 X, 80 ml) and ethyl ether (3 X, 80 ml). The silica gel was air dried for 6 h and then dried *in vacuo.*

B. The 5'-O-dimethoxytrityl and N-protected deoxynucleoside (2.5 mole) was dissolved in a solution of dry pyridine (5 ml) and N,N-dimethylaminopyridine (0.3 g). Succinic anhydride (2.0 mmole, 0.2 g) was added and the solution stirred at room temperature for 12 h. Thin layer chromatography (tlc) in acetonitrile:water (9:1, v/v) can be used to monitor the reaction. Unreacted nucleoside will have an $R_f$ of approximately 0.8 whereas the product will be a smear from $R_f$ 0.3 to $R_f$ 0.5. After completion of the reaction, solvent is removed in a rotary evaporator and the dry gum is redissolved in toluene (10 ml). Toluene is removed using a rotary evaporator and the toluene co-evaporation procedure is repeated. The dry gum free of pyridine and N,N-dimethylaminopyridine is dissolved in methylenechloride (30 ml). This solution is transferred to an extraction funnel and 10% ice-cold citric acid is added. After vigorous shaking and extraction, the organic phase is washed twice with water (15 ml ea) and then dried over sodium sulfate. Approximately 0.3 ml pyridine is added to the methylene chloride solution in order to minimize detritylation while drying over sodium sulfate. The methylene chloride solution is concentrated to 10 ml and the succinylated nucleoside isolated by precipitation into hexane:ether (1:1, v/v; 250 ml). The precipitate is collected by centrifugation and dried *in vacuo.*

To obtain the nitrophenyl esters, succinylated nucleoside (1 mmole) was dissolved in dry dioxane (3 ml) containing pyridine (0.3 ml). DCC (10 mmole, 0.22 g) and p-nitrophenol (0.143 g, 1 mmole) were added and the solution shaken for 2 h. Dicyclohexyl urea was removed by centrifugation. Analysis by tlc in acetonitrile:$H_2O$ (9:1, v/v) indicates the product with an $R_f$ of 0.8. This supernatant free of dicyclohexylurea is used directly for coupling to silica gel.

Silica gel prepared as outlined in A of this example, (5 g if 50 μ mole nucleoside/g desired; 2.5 g if 100μ mole nucleoside/g desired) was suspended in dry DMF. The p-nitrophenylsuccinylated nucleoside derivative (supernatant prepared herein) was added to the silica gel and the resulting suspension was shaken for two hours. An aliquot of silica gel (approx. 1 mg) was then removed for analysis. After washing the aliquot with DMF (2 X), methanol (3 X) and ethyl ether (2 X), 0.1 M toluenesulfonic acid in acetonitrile (1 ml) was added to the aliquot and the trityl released from silica as a red-orange color was observed. This analysis can be completed quantitatively if desired. If this analysis appears satisfactory (i.e. a positive trityl test), the bulk of the silica gel was washed with DMF (3 X, 10 ml ea), dioxane (3 X, 10 ml ea), methanol (5 X, 10 ml ea), and ethyl ether (3 X, 10 ml ea). Unreacted n-propylamino silyl groups were then blocked with a solution of acetic anhydride (0.7 ml) and dry pyridine (5 ml). The silica gel was isolated by centrifugation, decanting and repeated washing with methanol (4 X, 10 ml ea) and ethyl ether (2 S, 10 ml ea).

The assay for completeness of the capping or blocking of n-propylamino groups is as follows.

Take an aliquot (1 mg) of: (1) Underivatized Vydac-A, (2) Vydac derivatized with the aminopropyltriethoxysilane, (3) Vydac that has had nucleoside attached and subsequently blocked with acetic anhydride. Each sample was then treated with 250 μl of saturated sodium borate containing 0.2 mg/ml picryl sulfate. Vortex and centrifuge the reactant products. The underivatized Vydac should remain

white. The aminopropylsilyl Vydac should appear bright orange-red. The capped Vydac will be pale yellow-orange. This is probably due to interaction of picryl sulfate with ring nitrogens on nucleosides.

With some preparations, a contaminant of succinylated n-propylamino groups will result from the presence of succinic acid. This succinic acid may be present because all the succinic anhydride was not consumed during the succinylation or alternatively was not removed as succinic acid during the aqueous extraction with citric acid. If succinylated n-propylamino groups are present, they can be blocked in the following manner. The protected silica gel containing succinylated nucleoside (either 5 g or 2.5 g) was suspended in a solution of dry pyridine (5 ml) containing DCC (0.28 g) and p-nitrophenol (0.16 g) and shaken overnight at room temperature. Morpholine (0.2 ml) was then added and the suspension shaken for 10 minutes. Silica gel was isolated after centrifugation, decantation of the supernatant, and washing the silica gel with methanol (4 X, 10 ml ea), THF (3 X, 10 ml ea) and ethyl ether (3 X, 10 ml ea). After air drying, the silica gel was dried *in vacuo*.

A quantitative assay for the trityl cation and therefore the loading of nucleoside on the silica gel is as follows:

1. Weigh accurately approximately 1 mg of dry silica gel.
2. Add 1 ml of 0.1 M toluenesulfonic acid in acetonitrile.
3. Measure the absorbance at 498 nm. If the absorbance approaches 2.0, dilute and re-read. The loading can be calculated as follows:

$$\text{loading in } \mu\text{m moles/g} = \frac{(\text{Abs}^{498})\ (\text{dilution factor})}{\text{wt silica gel in mg}} \times 14.3$$

If 5 gm silica gel was used, the loading should be approximately 40 μ mole/g. If 2.5 gm silica gel was used, the loading will be approximately 100 μ mole/g.

In preferred embodiments, the amino groups, such as those on cytosine, adenine and guanine are protected. Protection of these groups is not a necessary part of this process but does enhance nucleoside solubilities in the appropriate solvents. Benzoyl, trityl (as previously defined herein) or isobutyryl groups provide suitable protecting groups, although other protecting groups can be used without altering this process. Protected nucleosides produced with good yields include 5′-O-dimethoxy-trityl-deoxythymidine [DMTrd(T)], 5′-O-dimethoxytrityl-N-benzoyldeoxycytidine (DMTrd(bzC)], 5′-O-dimethoxytrityl-N-benzoyl-deoxyadenosine (DMTrd(bzA)], and 5′-O-dimethoxytrityl-N-isobutyryldeoxyguanosine (DMTrd(ibG)] as protected nucleosides. A typical synthesis as illustrated with deoxyadenosine is as follows.

Example 3

This example illustrates the use of purine deoxynucleotides.

DMTrd(bzA) (.66 g, 1 mmole) in dry THF (3 ml) is added dropwise under an argon atmosphere to a stirred solution of the THF (3 ml) contianing methyldichlorophosphite (.113 ml, 1.2 mmole) and 2, 4, 6 trimethylpyridine (.633 ml, 4.8 mmole) at −78°C. After 10 minutes at −78°C, the reaction solution is filtered through a sintered glass funnel and solvent is removed by concentration *in vacuo.* Excess methyl phosphodichloridite is removed by dissolving the resulting gum in toluene: THF (2 ml, 2:1) and re-evaporating *in vacuo* to a gum. This procedure is repeated several times to insure removal of the dichloridite. The nucleoside phosphomonochloridite is converted to the tetrazolide. The gum resulting from the final re-evaporation is dissolved in THF (2 ml). A solution of tetrazole (.063 g, 0.9 mmole) in THF (2 ml) is then added dropwise with stirring at −78°C to the nucleoside phosphomonochloridite. After 10 minutes at −78°C, the solution is transferred to a centrifuge tube, spun at low speed, and the supernatant is removed. This solution contains the activated nucleoside methylphosphomonotetrazolide. If not used immediately, this tetrazolide can be placed in long term storage after precipitation by dropwise addition into dry pentane, followed by collection, drying *in vacuo,* and storing in sealed tubes under argon or other inert gas at −20°C. All operations are performed under inert gas to avoid oxidation. At no time is the active agent exposed to air.

The foregoing procedure is applicable for the preparation of activated thmidine, deoxycytidine, and deoxydenosine nucleotides. For the preparation of the activated deoxyguanosine nucleotide, the procedure is the same except for the stoichiometry. The molar ratio of DMTrd(ibG): methyldichlorophosphite; 2, 4, 6 trimethylpyridine and tetrazole is 1:0.9:3.8:0.7. The steps necessary for addition of one nucleotide to the modified silica gel polymer support follow. The removal of the dimethoxytrityl group from the nucleotide is accomplished by exposing the modified silica gel support to 0.1 M $ZnBr_2$ is nitromethane for 15 to 30 minutes. The support is then washed initially with butanol: 2, 6 lutidine: THF (4:1:5 by volume) and finally with THF. The solvent ratio is not important since this step is used to remove potential zinc esters of nucleosides. This step could be eliminated but lower yields may result. Other Lewis acids could be substituted for $ZnBr_2$, such as $BF_3$, $AlCl_3$ and $TiCl_4$. However $ZnBr_2$ is preferred. Protic acids can also be used. However approximately 3—5% depurination of each purine by protic acids is observed even when the amount of acid is reduced to the minimum amount needed to remove the dimethoxytrityl group. The next step in the process is condensation of the protected and activated nucleotide to the

nucleoside or oligonucleotide covalently bound to the support. This is accomplished by using 10—15 equivalents of the activated monotetrazolide and a reaction time of about one hour. The solvent is anhydrous THF. This process may also be used for the addition of the activated monochloridites, triazolides and nitroimidazolides. However, best results were obtained with the tetrazolide. The next step in the process is the blocking of unreated 5'-hydroxyl groups. This is accomplished using a solution of acetic anhydride, dimethylaminopyridine, pyridine and THF. This may also be accomplished using a 0.33 M solution of diethylmonotriazolephosphite in 2,6-lutidine/THF (1:5 by volume). The reaction time is 5 minutes and is followed by a THF wash. As a further alternative, a solution of phenylisocyanate/lutidine (45:55 by volume) and a 90 minute reaction time may be used for this step. This solution is then removed from the modified silica gel by washing the support with THF and with acetonitrile. The first procedure is preferred. This step can be eliminated or other reagents that react with 5'-hydroxyl groups and are compatible with the overall chemistry can be substituted therefore. However, by including this step, the final purification of the desirable oligonucleotide is rendered much easier. This is because the complexity of the total synthetic material bound to the support is reduced considerably. The final step in each cycle is oxidized of the phosphite to the phosphate. A composition of 0.1 $MI_2$ in water/2,6 lutidine/THF (1:1:3) is preferred, although other ratios can be used. Furthermore, other oxidizing agents such as N-chlorosuccinimide or aryl or alkyl peroxides could also be used. T-butyl peroxide is presently preferred as oxidizing agent. After the addition of the appropriate activated nucleotides in any predetermined sequence, the deoxyoligonucleotide is freed from protecting groups, isolated and characterized. The methyl group is removed from phosphotriesters using triethylammonium thiophenoxide in dioxane. The step is followed by treatment with concentrated $NH_4OH$ which removes amino protecting groups and frees the oligonucleotides from the support. The major product from each synthesis, as determined by high performance liquid chromatography, is found to be the desired oligonucleotide.

The compounds of formula I herein are new compounds which are particularly useful in forming the requisite phosphorus linkage, being more reactive and consequently more efficient than the corresponding prior art compounds of formula I wherein X is halogen. These compounds are readily prepared from the compounds in which X is halogen (as described, for example, in Example 3) or can be formed by reaction of a halo-(2' amino)-alkoxy-phosphine with the selected nucleotide.

The use of such heterocyclic aminophosphine compounds has been exemplified in Example 3 which illustrates the preparation of a tetrazolide and use thereof in forming the necessary phosphorus linkage. Employing this procedure, a variety of such compounds are prepared using tetrazole, nitroimidazole and triazole as well as various nucleosides to obtain the corresponding nucleoside phosphonomonamine. Particularly, such compounds include as nucleoside base thymine, cytosine, adenosine and guanine and such compounds are further protected with blocking groups as required, e.g. benzoyl groups on the amino groups of cytosine and adenine as well as isobutyryl on the amino group of guanine.

Example 4

The following example illustrates the use of purinedeoxynucleotides in the invention.

A. HPLC grade silica gel (2 g, Vydac TP—20, Separation Group, 100 $m^2/g$ surface area, 300 Å pore size, 20 μm particle size) was exposed to a 15% relative humidity atmosphere satd. LiCl) for at least 24 h. The silica (2.0 g) was then treated with 2-triethoxysilylpropylamine (2.3 g, 0.01 M in toluene for 12 h at 20° and 12 h at reflux under a Drierite drying tube. This reaction was completed on a shaking apparatus because magnetic stir bars pulverize the silica gel and should be avoided. The silica was isolated by centrifugation, washed successively (twice each) with toluene, methanol and ether and air dried.

B. The carboxylic acid group was introduced by agitating the silica so produced (2 g) and succinic anhydride (2.5 g, 0.025 M) in water. The pH was controlled (pH 2—6) by addition of 2 M NaOH. Completeness of the carboxylation reaction was qualitatively monitored using a picrate sulfate test. An aliquot of silica (approximately 2 mg) was treated with 0.5 ml of 0.1 M picrate sulfate in saturated sodium borate buffer (pH 10). The original silica reacted within 10 min and stained a bright yellow whereas the acylated product remained white. The succinic anhydride reaction was allowed to continue until the silica gel remained white during the picrate sulfate test. Usually the total reaction time was one hour and a second addition of succinic anhydride was required. After washing successively (twice each) with water, 0.1 M trichloroacetic acid, water, methanol and ether, compound 2 was air dried, dried *in vacuo,* and then treated with trimethylsilylchloride (1.25 ml, .01 M) in pyridine (7 ml) for 24 h at 25° and the product was then washed with methanol (4 times) and ether. Analysis for extent of carboxylation involved a two step procedure. An accurately weighed aliquot was treated with dicyclohexylcarbodiimide (DCC) and p-nitrophenol in pyridine. After several washings with tetrahydrofuran to remove unreacted p-nitrophenol, 10% piperidine in pyridine was added to the silica gel and the amount of p-nitrophenol released was measured at 410 nm using $1.57\times10^4$ as the extinction coefficient of p-nitrophenoxide. The incorporation of carboxylic acid was 200 μ mol/g.

C. The deoxynucleosides were joined to this product using DCC. 5'-O-dimethoxytritylthymidine (1.1 g, 2.16 mmol) DCC (2 g, 0.01 mol), and 2 (4 g, 0.8 mmol carboxylic acid) were agittated in dry pyridine (21 ml) for 2 days. O-Nitrophenol (1.4 g, 0.01 mol) was added, the mixture was agitated for an additional day, and then the reaction was quenched with morpholine (1 ml, .011 mol). After washing with methanol and ether, the silica gel was analyzed for unreacted carboxylic acid. Usually a second treatment with DCC (2 g, 0.01

mol) and p-nitrophenol (1.4 g, 0.01 mol) in dry pyridine (20 ml) and finally morpholine (1 ml) was necessary to completely block the trace amount of free carboxylic acid (<10 μmol/g) that remains from the first blocking procedure.

5'-O-Dimethoxytritylthymidine, 5'-O-dimethoxytrityl-N-benzoyldeoxycytidine, 5'-O-dimethoxytrityl-N-isobutyryl deoxyguanosine and 5'-O-dimethoxytrityl-N-benzoyldeoxyadenosine were converted to activated nucleoside by introduction of the requisite tetrazolide group using the following procedure.

5'-O-Dimethoxytritylthymidine (1.6 g, 2.9 mmol) in anhydrous tetrahydrofuran (5 ml) was added dropwise to a well stirred solution at −78° of $CH_3OPCl_2$ (0.33 ml, 2.6 mmol) and collidine (1.86 ml, 14.1 mmol) in anhydrous tetrahydrofuran (5 ml). A white precipitate formed during the addition. The mixture was stirred for 15 min at −78° and then filtered through a sintered glass funnel to remove collidine hydrochloride. The collidine hydrochloride was washed with dry tetrahydrofuran (1 ml). The filtrate was then diluted with dry toluene and concentrated to a gum. After dry argon had been bled into the apparatus, a solution (6 ml) containing toluene:tetrahydrofuran (2:1) was added and the gum was allowed to dissolve completely in this solution. Solvent was removed by concentation *in vacuo.* This reconstruction using a solution of toluene and tetrahydrofuran was repeated three times. After the final concentration, the gum was dissolved in dry tetrahydrofuran (3 ml), cooled to −78° and a solution of tetrazole (.18 g, 2.6 mmol) in dry tetrahydrofuran (3 ml) was added dropwise. A white precipitate of collidine hydrochloride formed during the addition. The mixture was stirred an additional 10 min at −78° and then transferred using positive argon pressure and a cannula to a centrifuge tube filled with argon. The supernatant recovered after centrifugation contained the tetrazolylphosphite product which can be used directly for synthesis of deoxyoligonucleotides. Alternatively, the tetrazolylphosphite can be stored as a precipitate and reconstituted as needed.

The aforesaid phosphites, i.e. activated nucleotides, were used in the synthesis of deoxyoligonucleotides in an automated apparatus in accordance with the accompanying drawing.

*Synthesis of deoxyolgonucleotides.* The apparatus consists of a Milton Roy Minipump, three way Altex slide valves, a recycle valve (a modified Altex valve) and an injector loop (a three way Altex valve). All connections were with teflon tubing and were designed to minimize the tubing volume in the recycle loop. The column was an 11 mm Ace glass column that had been shortened to approximately 1 ml capacity. Cellulose filters were used to support the silica bed. The filtered were acetylated with a solution of acetic anhydride and pyridine (1:1 based on volume) for 4 h at 50° before use. The total volume contained within the recycle loop of this apparatus was approximately 2.5 ml. The tetrahydrofuran reservoir was protected from air with a nitrogen bubbler and the $ZnBr_2$ solution was protected from moisture with the Drierite tube.

The various chemical operations that must be performance for the addition of one nucleotide to the silica are listed in Table II.

Table II Protocol for Machine Assisted Polynucleotide Synthesis

| Reagent or Solvent[a,b] | Time (min) | Machine Mode |
|---|---|---|
| Satd. $ZnBr_2/CH_3NO_2$ | 30 | Flush |
| $CH_3(CH_2)_2CH_2OH$/2,6-lutidine/THF | 5 | Flush |
| THF | 10 | Flush |
| Activated Nucleotide | 60 | Recycle |
| $(CH_3CH_2O)_2P$ (triazole) | 5 | Recycle |
| THF | 2 | Flush |
| $I_2$ Oxidation | 5 | Flush |
| THF | 5 | Flush |
| $CH_3NO_2$ | 3 | Flush |

[a]THF, Tetrahydrofuran

[b]A nitromethane solution saturated with $ZnBr_2$ is approximately 0.1 M in $ZnBr_2$

Typically, 0.25 g of 3 (10 μ mole thymidine) was loaded into the column and the silica washed with nitromethane. The 5'-O-dimethoxytrityl group was removed by flushing the column (30 min) with

nitromethane saturated with $ZnBr_2$ (approximately 0.1 M in $ZnBr_2$) at a pump speed of 1 ml/min. The completeness of deprotection was monitored visually or spectrophotometrically by observing the release of a bright orange dimethoxytrityl cation. By measuring the absorbance at 498 nm, the completeness of the previous condensation step was monitored. This step was followed successively by a wash with a solution of n-butanol:2,6-lutidine:tetrahydrofuran (4:1:5) for 5 min at a flow rate of 2 ml/min. The next step was a wash for 5 min (5 ml/min) with dry tetrahydrofuran. During the course of this washing step, the recycle valve and the injector port were also flushed with dry tetrahydrofuran and the effectiveness of this wash was monitored at 254 nm using a spectrophotometer. The condensation step was next completed using activated nucleotide that had been reconstituted using dry tetrahydrofuran. The reconstituted solution was stored in a dry ice/acetone bath over argon but condensation reactions were carried out at room temperature. When reconstituted, activated nucleotide stored in this way was stable for several days. Approximately 10 equivalents of activated nucleotide (100 μ mole for 0.25 g of 4) in 0.5 to 0.8 ml of tetrahydrofuran was injected into the apparatus and the machine switched to the recycle mode. The activated nucleotide was circulated through the silica gel for 1 h at a pump speed of 2 ml/min. Aliquots of activated nucleotide from the apparatus were then collected directly into dry methanol and water. Analysis as described previously indicated whether activated nucleotide was still present in the system. Usually this is the case. However, occasionally (approximately 1 in 10) the bis methyl phosphite of the deoxynucleotide was not observed by this essay. When this occurred, the condensation step was repeated to prevent the possibility of incomplete reaction. The next step involves capping unreacted 5′-O-hydroxyls by adding diethoxytriazoylphosphine (1 ml of a 0.3 M solution in tetrahydrofuran directly to the solution of activated nucleotide and continuing the recycle mode for 5 min at a pump speed of 2 ml/min. Residual activated nucleotide and the capping reagent were then flushed from the apparatus using dry tetrahydrofuran (2 min at 5 ml/min). This step was followed by oxidation of phosphites using a solution of tetrahydrofuran:2,6-lutidine:water (2:1:1) containing 0.2 M $I_2$. The solution was flushed through the apparatus for 5 min (2 ml/min). Finally the cycle was completed by flushing the system first with dry tetrahydrofuran for 3 min (5 ml/min) and nitromethane for 2 min (5 ml/min). This cycle is then repeated on appropriate number of times to complete the desired sequence.

*Isolation of Deoxyoligonucleotides.* The completely deprotected deoxyoligonucleotides were isolated by the following procedure. An aliquot (10 mg) of the silica gel containing the deoxyoligonucleotide triester in protected form was first treated with thiophenol:triethylamine:dioxane (1:1:2, v/v). After 45 min of gentle shaking, the silica gel was recovered by centrifugation and washed with methanol (4 times) and ethyl ether. After air drying, the deoxyoligonucleotide was removed from the support by a three hour treatment with concentrated ammonium hydroxide at 20° followed by centrifugation. Base protecting groups were removed by warming the supernatant at 50° for 12 h in a sealed tube. The 5′-O-dimethoxytrityldeoxyoligonucleotide was isolated by concentrating the hydrolysate *in vacuo,* dissolving the residue in 0.1 M triethylammonium acetate (pH 7.0) and chromatographing this material on a $C_{18}$ reverse phase hplc column (Waters Associates). The eluting buffer was 0.1 M triethylammonium acetate containing 26% acetonitrile. The peak containing 5′-O-dimethoxytrityldeoxyoligonucleotide was concentrated *in vacuo* and the residue was treated at 20° for 15 min with acetic acid-water (4:1, v/v) to remove the 5′-O-dimethoxytrityl group. The completely deprotected deoxyoligonucleotide was isolated by concentration of the acetic acid solution in vacuo, dissolving the residue in 25 mM triethylammonium bicarbonate (pH 7), the extraction of dimethoxytritanol with water saturated ether.

*Characterization of Deoxyoligonucleotides.* The 5′-hydroxyl of each deoxyoligonucleotide was phosphorylated using [5′—$^{32}$P]ATP and T4-kinase. The amount of deoxyoligonucleotide used in a phosphorylation reaction was determined by measuring the absorbance and using a calculated extinction coefficient which assumed no hypochromicity for the deoxyoligonucleotide. Phosphorylated deoxyoligonucleotides were separated from excess ATP by desalting on a G—50—40 Sephadex column using 10 mM triethylammonium bicarbonate (pH 7) as eluant. Gel electrophoresis on polyacrylamide and two dimension analysis were completed using standard procedures.

*Synthesis of d(C-G-T-C-A-C-A-A-T-A).* Silica gel modified with 5′-O-dimethoxytritylthymidine (0.25 g, 50 m/g) was loaded into the column and the cycle was started by washing the silica gel with nitromethane and removing the 5′-dimethoxytrityl group with $ZnBr_2$. Elongation was performed as previously described using an approximate tenfold excess of the incoming activated nucleoside phosphite (.1 mM) at each condensation. Synthesis was continued to the completion of the deoxyoctanucleotide, d(T-C-A-C-A-A-T-T). At this point the silica was divided into two approximately equal portions. One portion was elongated to the deoxydecanucleotide in standard fashion. The overall yield was 64% based on the amount of dimethoxytrityl group bound to the support and 30% was the yield isolated from a reverse phase hplc column.

*Synthesis of d(A-C-G-C-T-C-A-C-A-A-T-T).* The remaining portion of d(T-C-A-C-A-A-T-T) was elongated in standard fashion in the machine to the deoxydodecanucleotide, the overall yield was 55% based on the dimethoxytrityl group bound to the support. The isolated yield was not accurately determined.

The following oligonucleotides were prepared using the procedures described:

5'-d(A-A-T-T-C-A-C-C-G-T-G)

5'-d(C-G-T-G-T-T-G-A-C-T)

5'-d(A-T-T-T-T-A-C-C-T-C-T)

5'-d(G-G-C-G-G-T-G-A-T-A)

5'-d(A-T-G-A-G-C-A-C)

5'-d(A-A-T-T-G-T-G-C)

5'-d(T-C-A-T-T-A-T-C-A)

5'-d(C-C-G-C-C-A-G-A-G)

5'-d(G-T-A-A-A-A-T-A-G-T-C-A)

5'-d(A-C-A-C-G-C-A-C-G-G-T-G)

The procedures described in the foregoing examples can also be used for synthesizing mixed nucleoside and deoxynucleoside oligonucleotides by the simple expediency of inserting the desired nucleoside moiety at the desired point in the sequence. Thus, the present process, in addition to being useful for production of oligomucleotides of natural sequence of the individual nucleosides, can be used to produce synthetic oligonucleotides hitherto unknown in natural occurrence which can be useful in research and synthesis of polynucleotides, and eventually genes for use in biological systems.

A particularly preferred embodiment of the present invention is the detritylation of the 5'-O trityl nucleosides, deoxynucleotides, oligonucleotides, oligodeoxynucleotides, polynucleotides and polydeoxynucleotides using a Lewis acid, particularly zinc bromide, although other Lewis acids can also be employed, e.g. titanium tetrachloride. The use of Lewis acids is superior to the use of protic acids for removal of the trityl group from the 5"-O- position since reaction is extremely fast and is not accompanied by depurination. The process is also specific for 5'-O-trityl and thus provides a very practical procedure to permit production of 3'-O-trityl blocked nucleosides by reaction with the 3'-O- and 5'-O ditritylated compound.

The procedure merely requires contacting the reactants preferably in a reaction solvent and detritylation occurs within short reaction times. The Lewis acid is normally suspended in the reaction solvent and the solvent is usually water-free to prevent reaction with the Lewis acid to form protic acids. In present experience, nitromethane is the preferred solvent although a variety of other solvents can be used, e.g. dioxane and tetrahydrofuran and mixtures of such solvents with each other and other solvents such as acetone, methylene chloride, and the like.

The rate of detritylation was measured and comparative data is provided in Table IV.

Table III - The Rate of Detritylation and Depurination of 5'-Dimethoxytrityl-N-benzoyldeoxyadenosine Using Various Solutions*

| Solution | Temp. | Detritylation Time | Detritylation % | Depurination Time | Depurination % |
|---|---|---|---|---|---|
| satd. $ZnBr_2/CH_3NO_2$** | 18°C | <1 min | 100 | 10 hrs | 50 |
| satd. $ZnBr_2/CH_3NO_2$ | 0°C | 10 min | 100 | 21 hrs | <5 |
| 2% toluenesulfonic acid/ $CHCl_3:CH_3OH$ (7 : 3) | 18°C | <1 min | 100 | 5 min | 50 |
| 0.5% toluenesulfonic acid/$CHCl_3:CH_3OH$ (7 : 3) | 0°C | 10 min | 100 | 8 hrs | 50 |

*All depurination results summarized in this communication were obtained by analyzing reaction mixtures using reverse phase high pressure liquid chromatography. Estimates of detritylation times were by thin layer chromatography.

**A nitromethane solution saturated with $ZnBr_2$ is approximately 0.1 M in $ZnBr_2$.

The results obtained with various tritylthymidines at room temperature with zinc bromide is given in Table IV.

TABLE IV - The Rate of Detriylation and Degradation of 5'-Trityldeoxynucleosides Using $ZnBr_2$ at Room Temperature

| Deoxynucleoside | Detritylation Time | Detritylation % | Degradation Time |
|---|---|---|---|
| 5'-Dimethoxytrityl-N-isobutyryl deoxyguanosine | <1 min | 100 | 50 hrs |
| 5'-Dimethoxytrityl-N-benzoyldeoxycytosine | <1 min | 100 | 24 hrs |
| 5'-Dimethoxytritylthymidine | <1 min | 100 | 24 hrs |

and at 0°C. in Table III.

TABLE V - The Rate of Detritylation of Various Tritylthymidines Using Saturated $ZnBr_2$ in Nitromethane at 0°C.

| Nucleoside | Time | % Detritylation |
|---|---|---|
| 5'-Dimethoxytritylthymidine | 1 min | 50 |
| 5'-Monomethoxytritylthymidine | 1 min | 50 |
| 5'-Tritylthymidine | 10 min | 50 |
| 3'-Monomethoxytritylthymidine | 30 min | 10 |

The detritylation procedure is not restricted to polymer support synthesis but is also useful for strictly solution synthesis procedures where the use of trityl groups is included in the reaction sequence.

**Claims for the Contracting States: BE CH DE FR GB IT LI NL**

1. A process for preparing a modified inorganic polymer represented by the formula:

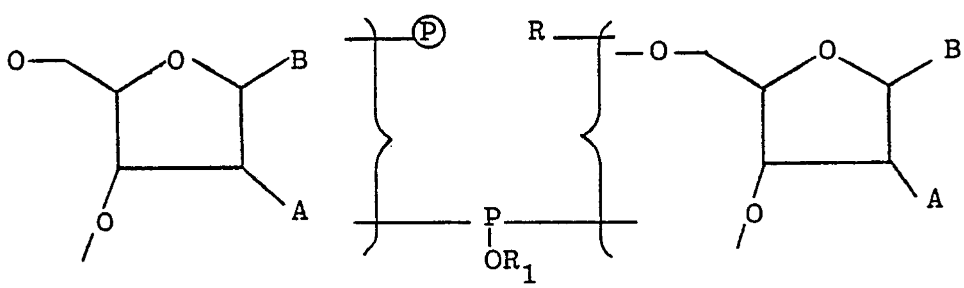

wherein Ⓟ is an inorganic polymer linked to the 3'- or 5'-O of the nucleoside through a hydrolyzable covalent bond; R is H or a blocking group; $R_1$ is a hydrocarbyl radical containing up to 10 carbon atoms; each B is a nucleoside or deoxynucleotide base; and each A is H or OR;
which process comprises condensing a compound of the formula:

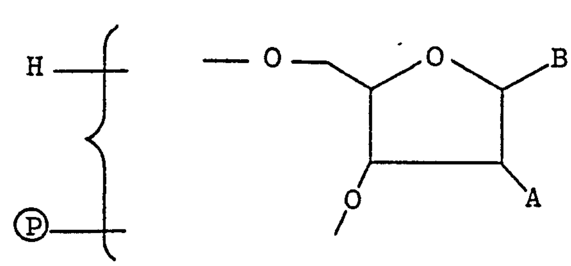

with a compound of the formula:

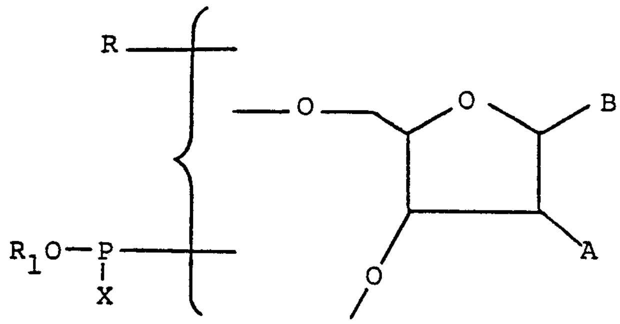

wherein A, B, Ⓟ, R and $R_1$ are as previously defined, and X is a secondary amino group attached through the amino nitrogen.

2. A process according to Claim 1, wherein a compound of the formula:

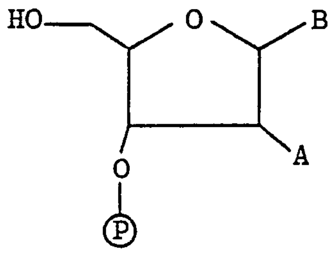

is condensed with a compound of the formula:

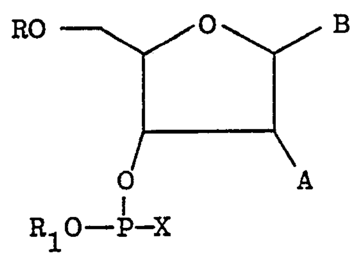

wherein A, B, Ⓟ, X, R and $R_1$ are as defined in Claim 1, to prepare a modified inorganic polymer of the formula:

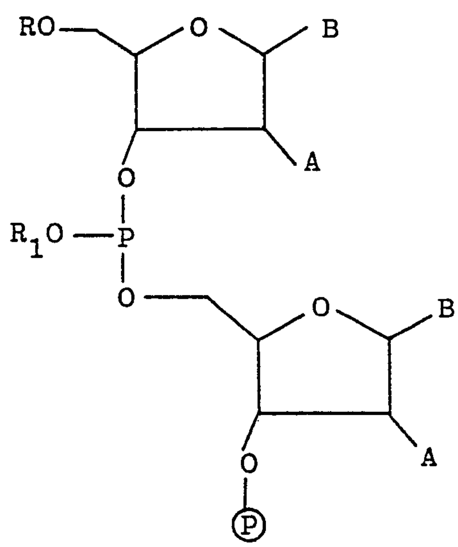

3. A process according to Claim 1 or Claim 2, wherein $R_1$ is lower alkyl.

4. A process according to any preceding claim, wherein A is H.

5. A process according to any preceding claim, wherein B is selected from adenine, guanine, cytosine, uracil and thymine.

6. A process according to any preceding claim, wherein Ⓟ is a silica gel.

7. A process according to Claim 6, wherein free silanol hydroxy groups on the silica gel are blocked.

8. A process according to Claim 6 or Claim 7, wherein the silica is macroporous and of a particle size ranging from 5 to 1000 µm.

9. A process according to any preceding claim, wherein X is a secondary amine group which is formed by the removal of the hydrogen atom from a ring nitrogen of a nitrogen heterocyclic compound which contains unsaturated bonds in the ring structure.

10. A process according to Claim 9, wherein said nitrogen heterocyclic compound is selected from tetrazole, triazole and nitroimidazole.

11. A process according to any preceding claim, wherein the 5'-O- blocking group is a trityl group.

12. A process according to Claim 11, wherein the trityl group is dimethoxytrityl or monomethoxytrityl.

13. A process according to Claim 6, or to any one of Claims 7—12 when appendant to Claim 6, wherein Ⓟ is linked to the 3'- or 5'-O of the nucleoside through a base hydrolysable covalent bond on the formula:

$$—(CH_2)_nNHCO—Z—CO—$$

wherein n is an integer from 1 to 5 and Z is a divalent hydrocarbyl radical.

14. A process of preparing a modified inorganic polymer of the formula:

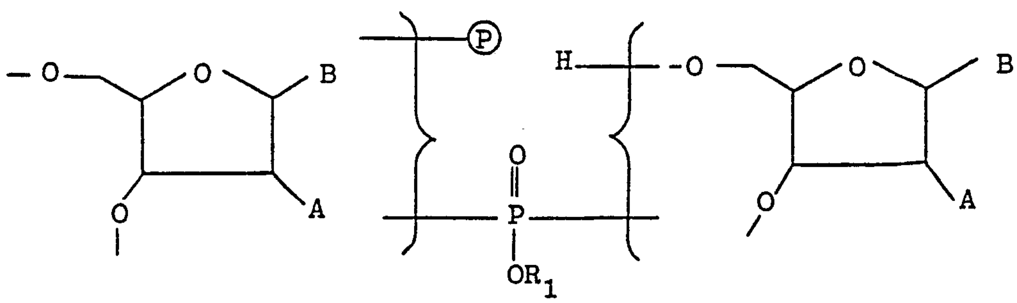

wherein Ⓟ, A, B, R and $R_1$ are as defined in Claim 1; which process comprises preparing a modified inorganic polymer by the process of any preceding claim, and thereafter oxidizing the phosphite linkage to a phosphate linkage, and removing the 3'-O- or 5'-O- blocking group of the product thus obtained.

15. A compound of the formula:

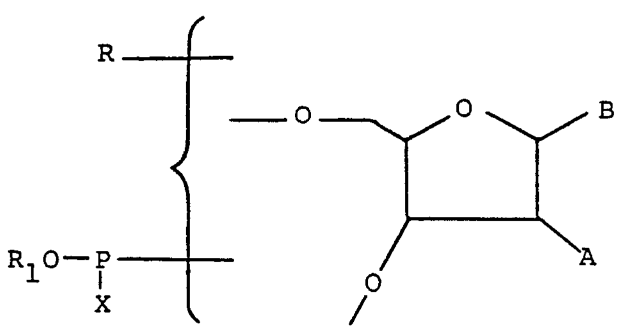

wherein B is a nucleoside or deoxynucleoside base; R is H or a blocking group, A is H or OR; R is a hydrocarbyl radical containing up to 10 carbon atoms; and X is a secondary amino group.

16. A compound of the formula:

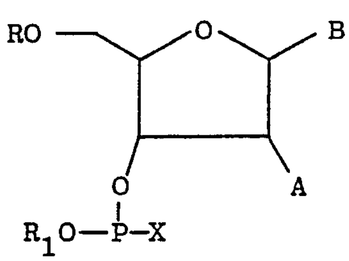

wherein B is a nucleoside or deoxynucleoside base; R is H or a blocking group; A is H or OR; $R_1$ is lower alkyl; and X is a secondary amino group formed by removal of the H atom of the secondary amino nitrogen of a nitrogen heterocyclic compound.

17. A compound according to Claim 16, wherein the secondary amino group is tetrazolyl, nitroimidazolyl or triazolyl.

18. A compound according to any one of Claims 16—17, wherein B is thymine, cytosine, adenine or guanine.

19. A compound according to any one of Claims 16—18, wherein R is a trityl group.

20. A compound according to any one of Claims 16—18, wherein R is di-p-anisylphenylmethyl or p-anisylphenylmethyl.

21. A process for the production of an oligonucleotide or polynucleotide, which comprises the steps of:

(1) condensing the 3'-OH or 5'-OH of a nucleoside or oligonucleotide covalently llinked to an inorganic polymer by a coupling agent through the 5'-O- or 3'-O, respectively, of said nucleoside or oligonucleotide with a phosphite compound of the formula (I):

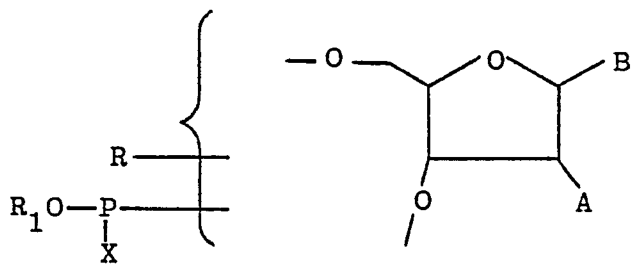

(I)

wherein R is a blocking group; B is a nucleoside or deoxynucleoside base; A is H or OR; $R_1$ is a hydrocarbyl radical containing up to 10 carbon atoms; and X is a secondary amino group; to form a product of the formula (II):

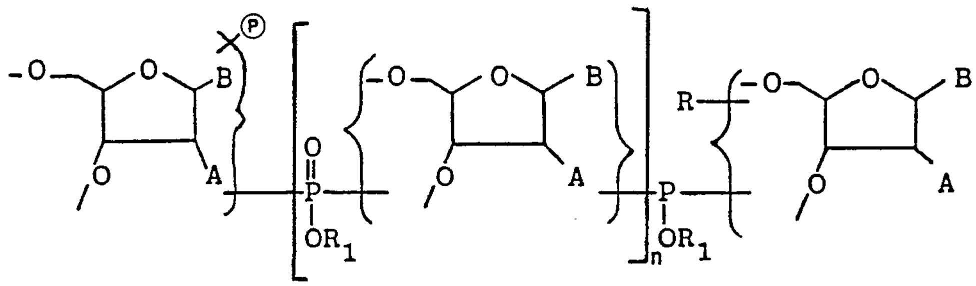

(II)

wherein Ⓟ represents the inorganic polymer, and n is an integer of at least one;

(2) oxidizing the terminal phosphite linkage of the product of step (1) to a phosphate linkage;

(3) removing the 3'-O- or 5'-O- blocking group of the product thus obtained;

(4) condensing the resulting product with a further nucleoside or oligonucleoside phosphite of formula (I), to form a product of formula (II) wherein n is an integer one greater than in the product of step (1);

(5) oxidizing the resulting phosphite triester to a phosphate triester;

(6) removing the 3'-O or 5'-O blocking group of the product thus obtained; and

(7) if desired repeating each of steps (4), (5) and (6) successively to add a desired sequence of nucleosides to the chain;

with the proviso that step (6) may be omitted following the addition of the final nucleoside to the chain.

22. A process according to Claim 27, wherein the phosphite compound of formula (I) is of the formula:

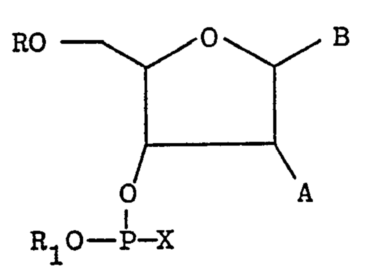

wherein R, $R_1$, X, A and B are as defined in Claim 15, and wherein said condensation occurs through the 5'-OH of said nucleoside or oligonucleotide.

23. A process according to Claim 21 or Claim 22, wherein $R_1$ is lower alkyl.

24. A process according to any one of Claims 21—23, wherein the inorganic polymer is a silica gel.

25. A process according to Claim 24, wherein the free silanol hydroxy groups on the silica gel are blocked prior to the condensation reaction of step (1).

26. A process according to Claim 24 or Claim 25, wherein Ⓟ is silica covalently bound to the nucleoside by the group —$Z_1$NHCOZCO— wherein Z and $Z_1$ are each alkylene of up to 5 carbon atoms.

27. A process according to any one of Claims 21—26, wherein A is H and R is a trityl blocking group.

28. A process according to Claim 27, wherein the trityl group is dimethoxytrityl.

29. A process according to any one of Claims 21—28, wherein step (2) and/or (5) are conducted using $I_2$ as the oxidizing agent.

30. A process according to any one of Claims 21—29, wherein step (3) and/or (6) are conducted by reaction with a Lewis acid.

31. A process according to Claim 20, wherein the Lewis acid is zinc bromide.

32. A process according to any one of Claims 21—31, including the further step (8) of hydrolyzing the phosphate triester product to form the oligonucleotide or polynucleotide and the inorganic polymer and separating the oligonucleotide or polynucleotide therefrom.

**Claims for the Contracting State: AT**

1. A process for preparing a modified inorganic polymer represented by the formula:

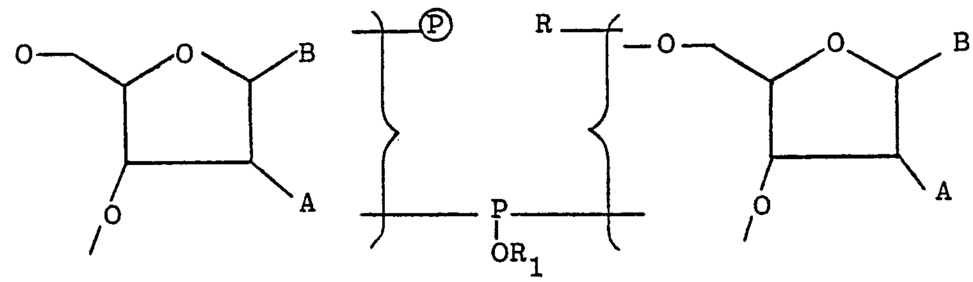

wherein Ⓟ is an inorganic polymer linked to the 3'- or 5'-O of the nucleoside through a hydrolyzable covalent bond; R is H or a blocking group; $R_1$ is a hydrocarbyl radical containing up to 10 carbon atoms; each B is a nucleoside or deoxynucleotide base; and each A is H or OR;
which process comprises condensing a compound of the formula:

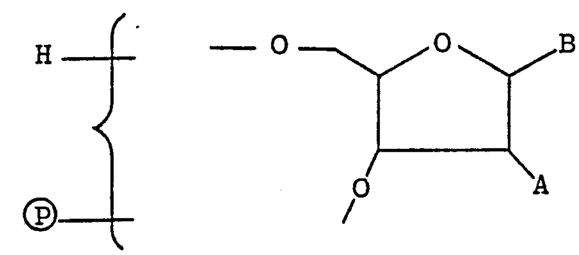

with a compound of the formula:

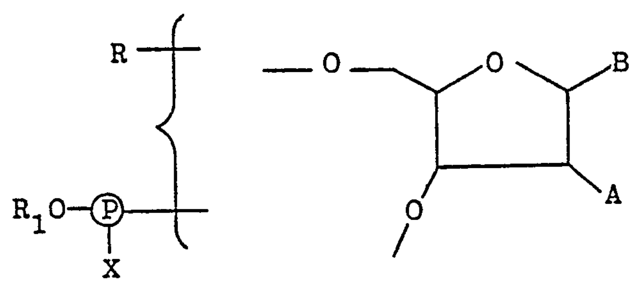

wherein A, B, Ⓟ, R and $R_1$ are as previously defined, and X is a secondary amino group attached through the amino nitrogen.

2. A process according to Claim 1, wherein a compound of the formula:

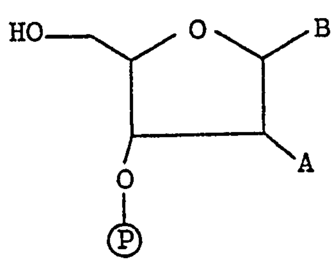

is condensed with a compound of the formula:

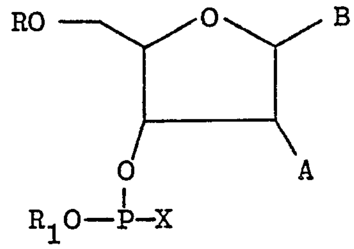

wherein A, B, Ⓟ, X, R and $R_1$ are as defined in Claim 1, to prepare a modified inorganic polymer of the formula:

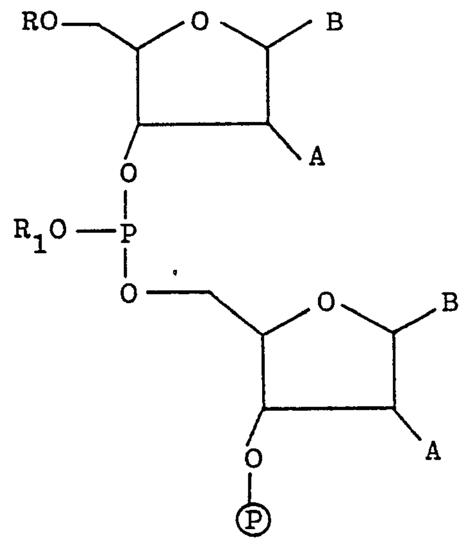

3. A process according to Claim 1 or Claim 2, wherein $R_1$ is lower alkyl.

4. A process according to any preceding claim, wherein A is H.

5. A process according to any preceding claim, wherein B is selected from adenine, guanine, cytosine, uracil and thymine.

6. A process according to any preceding claim, wherein Ⓟ is a silica gel.

7. A process according to Claim 6, wherein free silanol hydroxy groups on the silica gel are blocked.

8. A process according to Claim 6 or Claim 7, wherein the silica is macroporous and of a particle size ranging from 5 to 1000 μm.

9. A process according to any preceding claim, wherein X is a secondary amine group which is formed by the removal of the hydrogen atom from a ring nitrogen of a nitrogen heterocyclic compound which contains unsaturated bonds in the ring structure.

10. A process according to Claim 9, wherein said nitrogen heterocyclic compound is selected from tetrazole, triazole and nitroimidazole.

11. A process according to any preceding claim, wherein the 5'-O- blocking group is a trityl group.

12. A process according to Claim 11, wherein the trityl group is dimethoxytrityl or monomethoxytrityl.

13. A process according to Claim 6, or to any one of Claims 7—12 when appendant to Claim 6, wherein Ⓟ is linked to the 3'- or 5'-O of the nucleoside through a base hydrolysable covalent bond of the formula:

$$—(CH_2)_nNHCO—Z—CO—$$

wherein n is an integer from 1 to 5 and Z is a divalent hydrocarbyl radical.

14. A process of preparing a modified inorganic polymer of the formula:

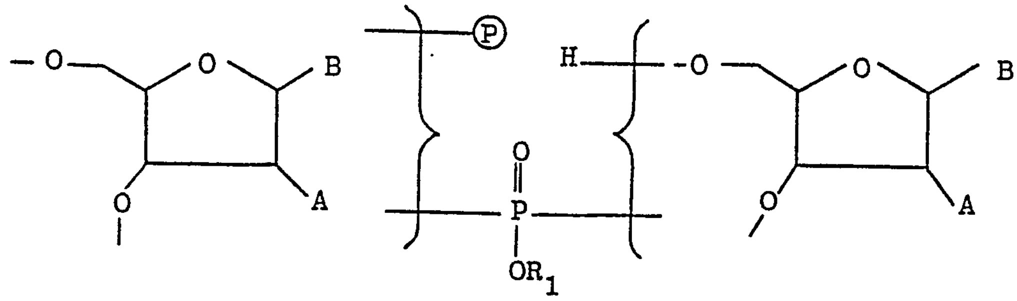

wherein Ⓟ, A, B, R and $R_1$ are as defined in Claim 1; which process comprises preparing a modified inorganic polymer by the process of any preceding claim, and thereafter oxidizing the phosphite linkage to a phosphate linkage, and removing the 3'-O- or 5'-O- blocking group of the product thus obtained.

15. A process for the production of an oligonucleotide or polynucleotide, which comprises the steps of:

(1) condensing the 3'-OH or 5'-OH of a nucleoside or oligonucleotide covalently linked to an inorganic polymer by a coupling agent through the 5'-O- or 3'-O, respectively, of said nucleoside or oligonucleotide with a phosphite compound of the formula (I):

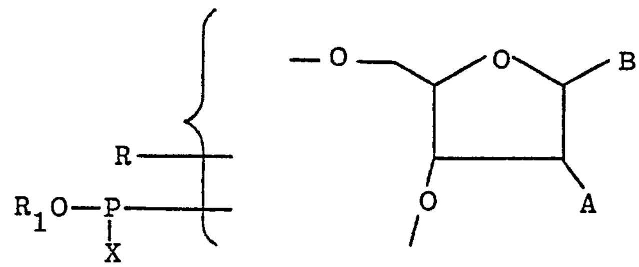

(I)

wherein R is a blocking group; B is a nucleoside or deoxynucleoside base; A is H or OR; $R_1$ is a hydrocarbyl radical containing up to 10 carbon atoms; and X is a secondary amino group; to form a product of the formula (II):

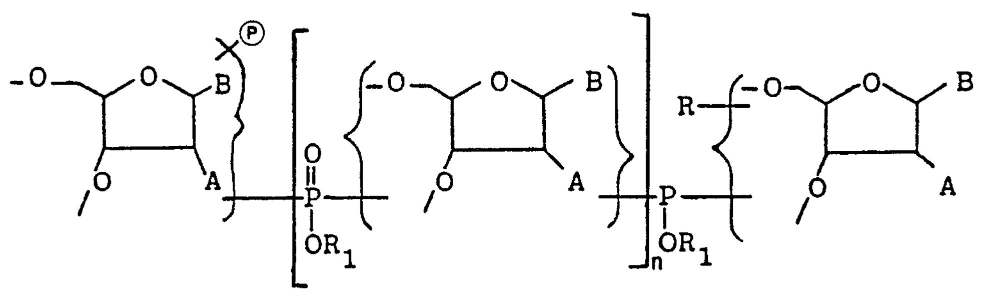

(II)

wherein Ⓟ represents the inorganic polymer, and n is an integer of at least one;

(2) oxidizing the terminal phosphite linkage of the product of step (1) to a phosphate linkage;

(3) removing the 3'-O- or 5'-O- blocking group of the product thus obtained;

(4) condensing the resulting product with a further nucleoside or oligonucleoside phosphite of formula (I), to form a product of formula (II) wherein n is an integer one greater than in the product of step (1);

(5) oxidizing the resulting phosphite triester to a phosphate triester;

(6) removing the 3'-O or 5'-O blocking group of the product thus obtained; and

(7) if desired repeating each of steps (4), (5) and (6) successively to add a desired sequence of nucleosides to the chain;

with the proviso that steps (5) and (6) may be omitted following the addition of the final nucleoside to the chain.

16. A process according to Claim 15, wherein the phosphite compound of formula (1) is of the formula:

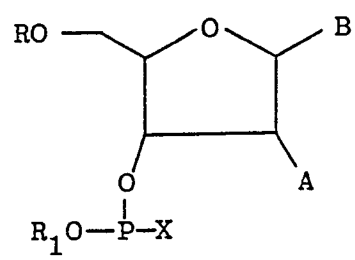

wherein R, $R_1$, X, A and B are as defined in Claim 15, and wherein said condensation occurs through the 5'-OH of said nucleoside or oligonucleotide.

17. A process according to Claim 15 or Claim 16, wherein $R_1$ is lower alkyl.

18. A process according to any one of Claims 15—17, wherein the inorganic polymer is a silica gel.

19. A process according to Claim 18, wherein the free silanol hydroxy groups on the silica gel are blocked prior to the condensation reaction of step (1).

20. A process according to Claim 18 or Claim 19, wherein Ⓟ is silica covalently bound to the nucleoside by the group $-Z_1NHCOZCO-$ wherein Z and $Z_1$ are each alkylene of up to 5 carbon atoms.

21. A process according to any one of Claims 15—20, wherein A is H and R is a trityl blocking group.

22. A process acording to Claim 21, wherein the trityl group is dimethoxytrityl.

23. A process according to any one of Claims 15—22, wherein step (2) and/or (5) are conducted using $I_2$ as the oxidizing agent.

24. A process according to any one of Claims 15—23, wherein step (3) and/or (6) are conducted by reaction with a Lewis acid.

25. A process according to Claim 24, wherein the Lewis acid is zinc bromide.

26. A process according to any one of Claims 15—25, including the further step (8) of hydrolyzing the phosphate triester product to form the oligonucleotide or polynucleotide and the inorganic polymer and separating the oligonucleotide or polynucleotide therefrom.

27. The use in a process for the synthesis of a longer chain oligonucleotide or polynucleotide of a compound of the formula:

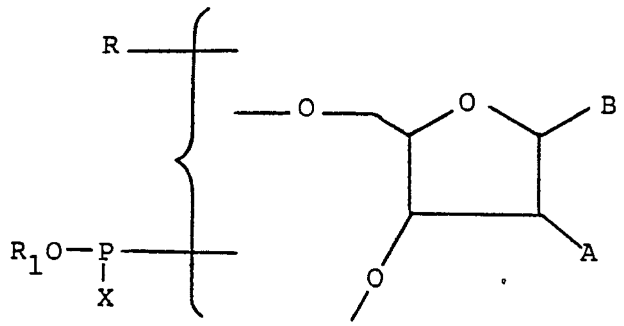

wherein B is a nucleotide or deoxynucleoside base; R is H or a blocking group, A is H or OR; $R_1$ is a hydrocarbyl radical containing up to 10 carbon atoms; and X is a secondary amino group.

28. The invention according to Claim 27, wherein said compound has the formula:

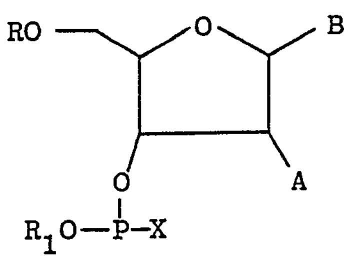

wherein B is a nucleoside or deoxynucleoside base; R is H or a blocking group; A is H or OH ; $R_1$ is lower alkyl; and X is a secondary amino group formed by removal of the H atom of the secondary amino nitrogen of a nitrogen heterocyclic compound.

29. The invention according to Claim 28, wherein the secondary amino group is tetrazolyl, nitroimidazolyl or triazolyl.

30. The invention according to any one of Claims 27—29, wherein B is thymine, cytosine, adenine or quanine.

31. The invention according to any one of Claims 27—30, wherein R is a trityl group.

32. The invention according to any one of Claims 27—30 wherein R is di-p-anisylphenylmethyl or p-anisylphenylmethyl.

33. The invention according to any one of Claims 27—32, wherein A is H.

**Patentansprüche für die Vertragsstaaten: BE CH DE FR GB IT LI NL**

1. Verfahren zur Herstellung eines modifizierten anorganischen Polymers der Formel

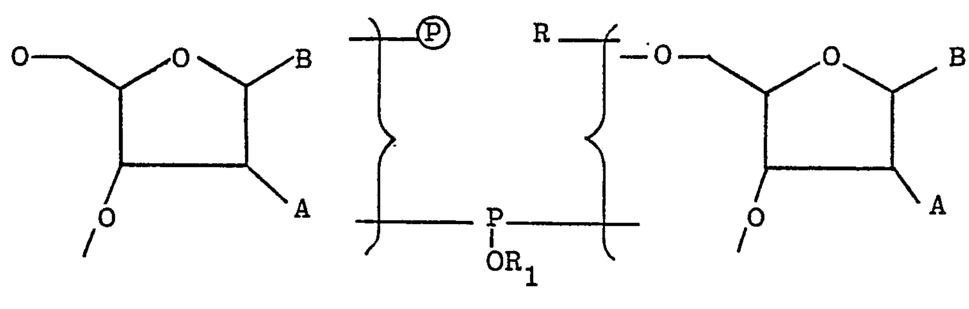

worin Ⓟ ein anorganisches Polymer ist, das an den 3'-O oder 5'-O des Nucleosides über eine hydrolysierbare kovalente Bindung gebunden ist; R für H oder eine Blockierungsgruppe steht; $R_1$ ein Hydrocarbylrest mit bis zu 10 C-Atomen ist; jedes B eine Nucleosid- oder Desoxynucleosidbase darstellt; und jedes A für H oder OR steht;
welches Verfahren das Kondensieren einer Verbindung der Formel

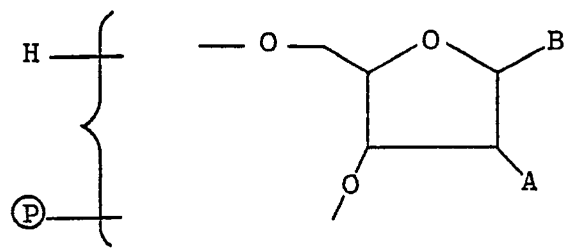

mit einer Verbindung der Formel

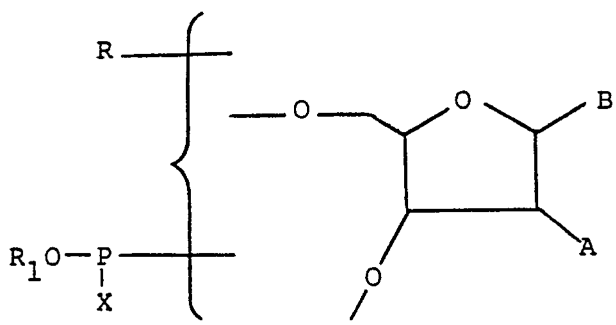

umfaßt, worin A, B, Ⓟ, R und $R_1$ die vorstehend angeführte Bedeutung besitzen und X eine sekundäre Aminogruppe darstellt, die über den Aminostickstoff gebunden ist.

2. Verfahren nach Anspruch 1, worin eine Verbindung der Formel

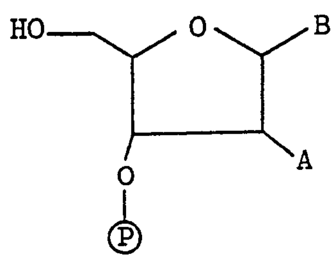

mit einer Verbindung der Formel

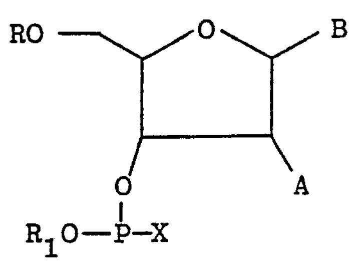

worin A, B, Ⓟ, X, R und $R_1$ die in Anspruch 1 angeführte Bedeutung besitzen kondensiert wird, um ein modifiziertes anorganisches Polymer der Formel

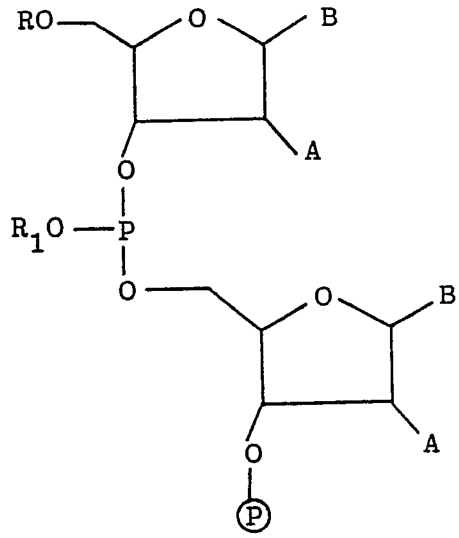

zu erhalten.

3. Verfahren nach Anspruch 1 oder 2, worin $R_1$ Niederalkyl bedeutet.

4. Verfahren nach einem der vorhergehenden Ansprüche, worin A für H steht.

5. Verfahren nach einem der vorhergehenden Ansprüche, worin B aus Adenin, Guanin, Cytosin, Uracil und Thymin gewählt ist.

6. Verfahren nach einem der vorhergehenden Ansprüche, worin Ⓟ ein Silikagel ist.

7. Verfahren nach Anspruch 6, worin freie Silanolhydroxygruppen am Silikagel blockiert werden.

8. Verfahren nach Anspruch 6 oder 7, worin das Silikagel makroporös ist und Korngrößen von 5 bis 1000 µm aufweist.

9. Verfahren nach einem der vorhergehenden Ansprüche, worin X für eine sekundäre Aminogruppe steht, die durch Entfernung des Wasserstoffatoms aus einem Ringstickstoff einer heterocyclischen Stickstoffverbindung gebildet wird, die ungesättigte Bindungen in der Ringstruktur enthält.

10. Verfahren nach Anspruch 9, worin die genannte heterocyclische Stickstoffverbindung aus Tetrazol, Triazol und Nitroimidazol gewählt ist.

11. Verfahren nach einem der vorhergehenden Ansprüche, worin die 5'-O-Blockierungsgruppe eine Tritylgruppe ist.

12. Verfahren nach Anspruch 11, worin die Tritylgruppe Dimethoxytrityl oder Monomethoxytrityl ist.

13. Verfahren nach Anspruch 6 oder einem der Ansprüche 7 bis 12, sofern sie auf Anspruch 6 rückbezogen sind, worin P an den 3'-O oder 5'-O des Nucleosides durch eine basenhydrolysierbare kovalente Bindung der Formel

$$-(CH_2)_nNHCO-Z-CO-$$

gebunden ist, worin n eine ganze Zahl von 1 bis 5 darstellt und Z ein zweiwertiger Hydrocarbylrest ist.

14. Verfahren zur Herstellung eines modifizierten anorganischen Polymers der Formel

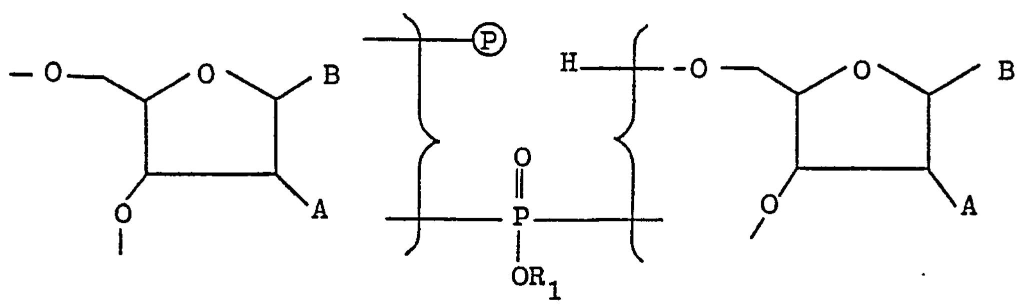

worin Ⓟ, A, B, R und $R_1$ die in Anspruch 1 angeführte Bedeutung besitzen; welches Verfahren die Herstellung eines modifizierten anorganischen Polymers nach einem Verfahren nach einem der vorhergehenden Ansprüche und das darauffolgende Oxydieren der Phosphitbindung zu einer Phosphatbindung und die Entfernung der 3'-O— oder 5'-O— Blockierungsgruppe aus dem so erhaltenen Produkt umfaßt.

15. Verbindung der Formel

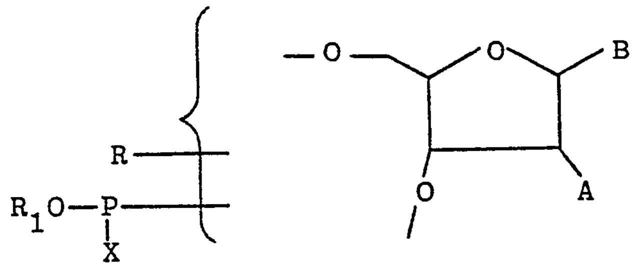

worin B für eine Nucleosid- oder Desoxynucleosidbase steht; R H oder eine Blockierungsgruppe bedeutet; A H oder OR ist; $R_1$ einen Hydrocarbylrest mit bis zu 10 C-Atomen bedeutet; und X eine sekundäre Aminogruppe darstellt.

16. Verbindung der Formel

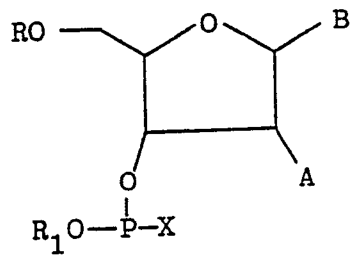

worin B eine Nucleosid- oder Desoxynucleosidbase ist; R H oder eine Blockierungsgruppe bedeutet; A für H oder OR steht; $R_1$ Niederalkyl ist; und X eine sekundäre Aminogruppe darstellt, die durch Entfernung des H-Atoms aus dem sekundären Aminostickstoff einer heterocyclischen Stickstoffverbindung gebildet ist.

17. Verbindung nach Anspruch 16, worin die sekundäre Aminogruppe Tetrazolyl, Nitroimidazolyl oder Triazolyl ist.

18. Verbindung nach Anspruch 16 oder 17, worin B für Thymin, Cytosin, Adenin oder Guanin steht.

19. Verbindung nach einem der Ansprüche 16 bis 18, worin R eine Tritylgruppe darstellt.

20. Verbindung nach einem der Ansprüche 16 bis 18, worin R Di-p-Anisylphenylmethyl oder p-Anisylphenylmethyl bedeutet.

21. Verfahren zur Herstellung eines Oligonucleotides oder Polynucleotides, welches die Schritte

(1) Kondensieren des 3'-OH oder 5'-OH eines Nucleosides oder oligonucleotides, das kovalent an ein anorganisches Polymer mittels eines Kopplungsmittels über den 5'-0 bzw. 3'-O des genannten Nucleosides oder Oligonucleotides gebunden ist, mit einer Phosphitverbindung der Formel (I):

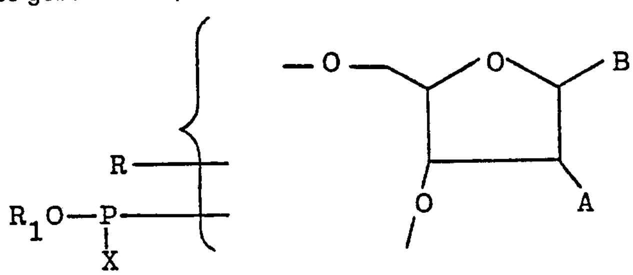

(I)

worin R eine Blockierungsgruppe ist; B eine Nucleosid- oder Desoxynucelosidbase darstellt; A für H oder OR steht; $R_1$ einen Hydrocarbylrest mit bis zu 10 C-Atomen bedeutet; und X eine sekundäre Aminogruppe ist; um ein Produkt der Formel (II)

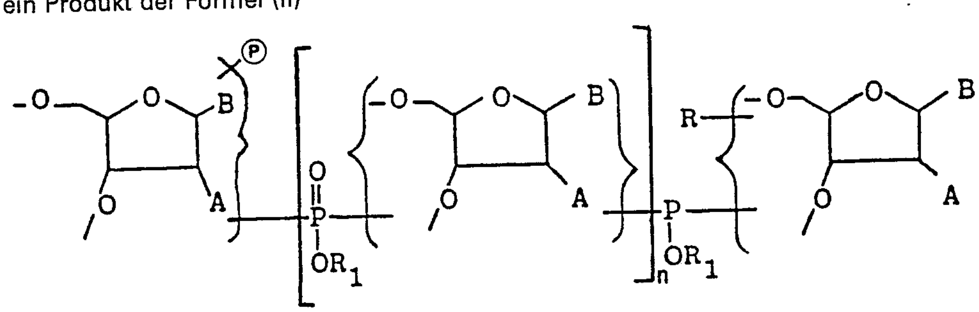

(II)

zu bilden, worin P das anorganische Polymer darstellt und n eine ganze Zahl von wenigstens eins ist;

(2) Oxydieren der endständigen Phosphitbindung des Produktes aus Schritt (1) zu einer Phosphatbindung;

(3) Entfernen der 3'-O— oder 5'-O—Blockierungsgruppe aus dem so erhaltenen Produkt;

(4) Kondensieren des entstandenen Produktes mit einem weiteren Nucleosid- oder Oligonucleosidphosphit der Formel (I), um ein Produkt der Formel (II) zu bilden, worin n eine ganze Zahl ist, die um einen Wert größer ist als die im Produkt nach Schritt (1);

(5) Oxydieren des entstandenen Phosphittriesters zu einem Phosphattriester;

(6) Entfernen der 3'-O— oder 5'-O—Blockierungsgruppe aus dem so erhaltenen Produkt; und

(7) nach Bedarf Wiederholen jedes der Schritte (4), (5) und (6) aufeinanderfolgend, um der Kette eine gewünschte Sequenz von Nucleosiden hinzuzufügen; mit der Maßgabe, daß Schritt (6) nach der Zugabe des letzten Nucleosides zur Kette weggelassen werden kann.

22. Verfahren nach Anspruch 21, worin die Phosphitverbindung der Formel (1) die Formel

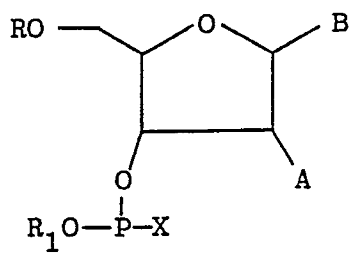

aufweist, worin R, $R_1$, X, A und B die in Anspruch 15 angeführte Bedeutung besitzen und worin die genannte Kondensation über das 5'OH des genannten Nucleosides oder Oligonucleotides erfolgt.

23. Verfahren nach Anspruch 21 oder 22, worin $R_1$ Niederalkyl bedeutet.

24. Verfahren nach einem der Ansprüche 21 bis 23, worin das anorganische Polymer ein Silikagel ist.

25. Verfahren nach Anspruch 24, worin die freien Silanohydroxygruppen am Silikagel vor der Kondensationsreaktion von Schritt (1) blockiert werden.

26. Verfahren nach Anspruch 24 oder 25, worin Ⓟ ein durch die Gruppe —$Z_1$NHCOZCO kovalent an das Nucleosid gebundenes Silika darstellt, worin Z und $Z_1$ jeweils Alkylen mit bis zu 5 C-Atomen bedeuten.

27. Verfahren nach einem der Ansprüche 21 bis 26, worin A für H steht und R eine Tritylblockierungsgruppe bedeutet.

28. Verfahren nach Anspruch 27, worin die Tritylgruppe Dimethoxytrityl ist.

29. Verfahren nach einem der Ansprüche 21 bis 28, worin Schritt (2) und/oder (5) unter Verwendung von $I_2$ als Oyxdationsmittel durchgeführt wird.

30. Verfahren nach einem der Ansprüche 21 bis 29, worin Schritt (3) und/oder (6) durch Reaktion mit einer Lewissäure durchgeführt, wird.

31. Verfahren nach Anspruch 30, worin die Lewissäure Zinkbromid ist.

32. Verfahren nach einem der Ansprüche 21 bis 31, umfassend den weiteren Schritt (8) des Hydrolysierens des Phosphattriesterproduktes, um das Oligonucleotid oder Polynucleotid und das anorganische Polymer zu bilden und das Oligonucleotid oder Polynucleotid daraus zu entfernen.

**Patentansprüche für den Vertragsstaat: AT**

1. Verfahren zur Herstellung eines modifizierten anorganischen Polymers der Formel

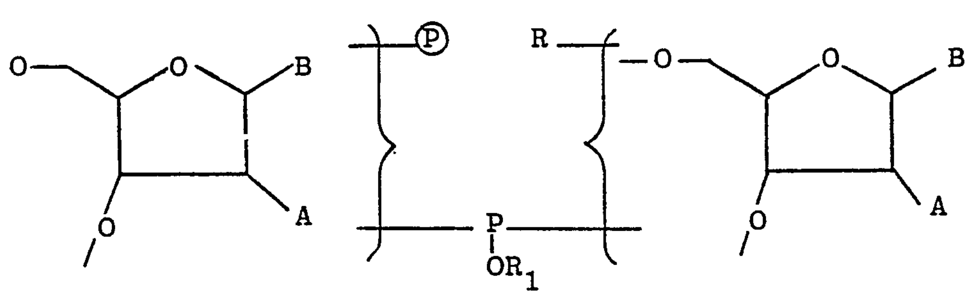

worin Ⓟ ein anorganisches Polymer ist, das an den 3'-O oder 5'-O des Nucleosides über eine hydrolysierbare kovalente Bindung gebunden ist; R für H oder eine Blockierungsgruppe steht; $R_1$ ein Hydrocarbylrest mit bis zu 10 C-Atomen ist; jedes B eine Nucleosid- oder Desoxynucleosidbase darstellt; und jedes A für H oder OR steht;

welches Verfahren das Kondensieren einer Verbindung der Formel

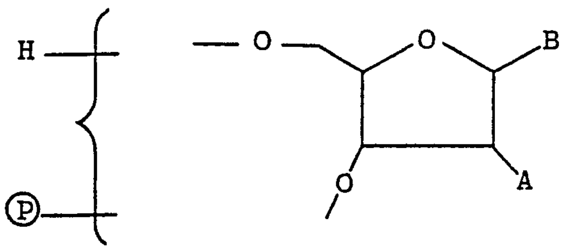

mit einer Verbindung der Formel

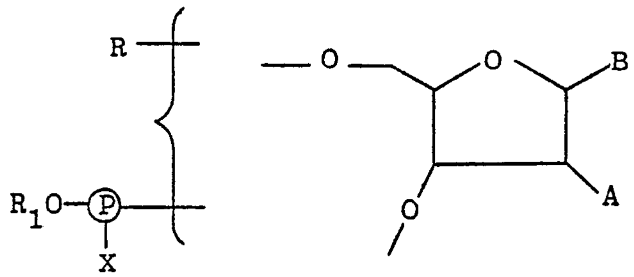

umfaßt, worin A, B, Ⓟ, R und $R_1$ die vorstehend angeführte Bedeutung besitzen und X eine sekundäre Aminogruppe darstellt, die über den Aminostickstoff gebunden ist.

2. Verfahren nach Anspruch 1, worin eine Verbindung der Formel

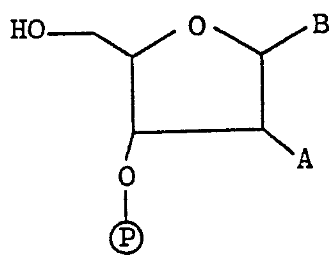

mit einer Verbindung der Formel

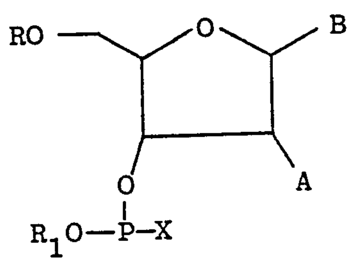

worin A, B, Ⓟ, X, R und $R_1$ die in Anspruch 1 angeführte Bedeutung besitzen, kondensiert wird, um ein modifiziertes anorganisches Polymer der Formel

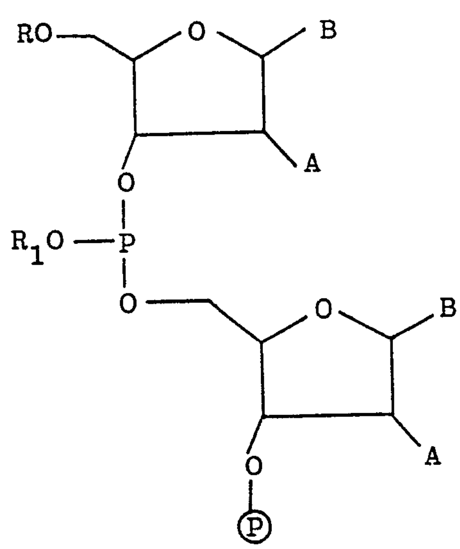

zu erhalten.

3. Verfahren nach Anspruch 1 oder 2, worin $R_1$ Niederalkyl bedeutet.

4. Verfahren nach einem der vorhergehenden Ansprüche, worin A für H steht.

5. Verfahren nach einem der vorhergehenden Ansprüche, worin B aus Adenin, Guanin, Cytosin, Uracil und Thymin gewählt ist.

6. Verfahren nach einem der vorhergehenden Ansprüche, worin Ⓟ ein Silikagel ist.

7. Verfahren nach Anspruch 6, worin freie Silanolhydroxygruppen am Silikagel blockiert werden.

8. Verfahren nach Anspruch 6 oder 7, worin das Silikagel makroporös ist und Korngrößen von 5 bis 1000 μm aufweist.

9. Verfahren nach einem der vorhergehenden Ansprüche, worin X für eine sekundäre Aminogruppe steht, die durch Entfernung des Wasserstoffatoms aus einem Ringstickstoff einer heterocyclischen Stickstoffverbindung gebildet wird, die ungesättigte Bindungen in der Ringstruktur enthält.

10. Verfahren nach Anspruch 9, worin die genannte heterocyclische Stickstoffverbindung aus Tetrazol, Triazol und Nitroimidazol gewählt ist.

11. Verfahren nach einem der vorhergehenden Ansprüche, worin die 5'-O-Blockierungsgruppe eine Tritylgruppe ist.

12. Verfahren nach Anspruch 11, worin die Tritylgruppe Dimethoxytrityl oder Monomethoxytrityl ist.

13. Verfahren nach Anspruch 6 oder einem der Ansprüche 7 bis 12, sofern sie auf Anspruch 6 rückbezogen sind, worin Ⓟ an den 3'-O oder 5'-O des Nucleosides über eine basenhydrolysierbare konvalente Bindung der Formel

$$—(CH_2)_nNHCO—Z—CO—$$

gebunden ist, worin n eine ganze Zahl von 1 bis 5 darstellt und Z ein zweiwertiger Hydrocarbylrest ist.

14. Verfahren zur Herstellung eines modifizierten anorganischen Polymers der Formel

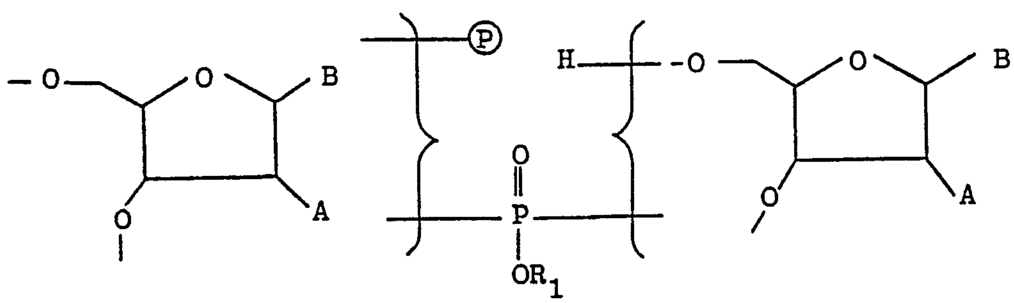

worin Ⓟ, A, B, R und $R_1$ die in Anspruch 1 angeführte Bedeutung besitzen; welches Verfahren die Herstellung eines modifizierten anorganischen Polymers nach einem Verfahren nach einem der Vorhergehenden Ansprüche und das darauffolgende Oxydieren der Phosphitbindung zu einer Phosphatbindung und die Entfernung der 3'-O— oder 5'-O—Blockierungsgruppe aus dem so erhaltenen Produkt umfaßt.

15. Verfahren zur Herstellung eines Oligonucleotides oder Polynucleotides, welches die Schritte

(1) Kondensieren des 3'-OH oder 5'-OH eines Nucleosides oder oligonucleotides, das kovalent an ein anorganisches Polymer mittels eines Kopplungsmittels über den 5'-0 bzw. 3'-O des genannten Nucleosides oder Oligonucleotides gebunden ist, mit einer Phosphitverbindung der Formel (I):

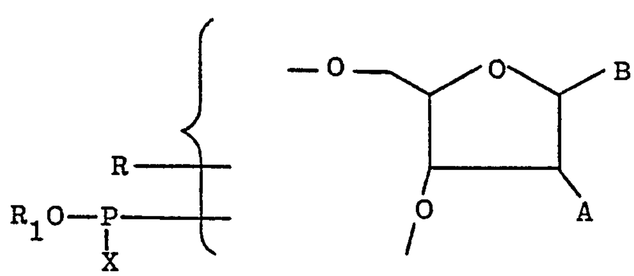

(I)

worin R eine Blockierungsgruppe ist; B eine Nucleosid- oder Desoxynucelosidbase darstellt; A für H oder OR stet; $R_1$ einen Hydrocarbylrest mit bis zu 10 C-Atomen bedeutet; und X eine sekundäre Aminogruppe ist; um ein Produkt der Formel (II)

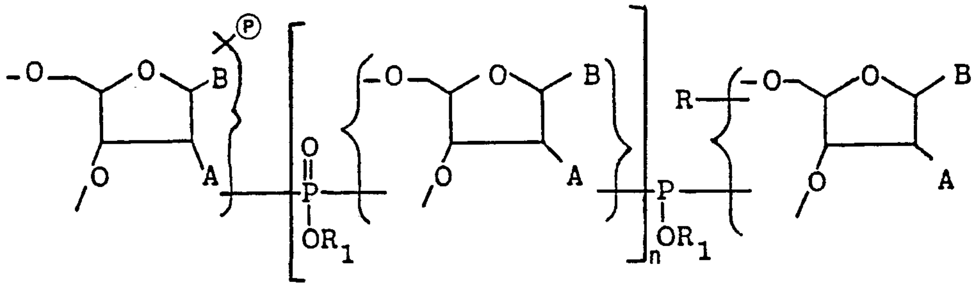

(II)

zu bilden, worin P das anorganische Polymer darstellt und n eine ganze Zahl von wenigstens eins ist;

(2) Oxydieren der endständigen Phosphitbindung des Produktes aus Schritt (1) zu einer Phosphatbindung;

(3) Entfernen der 3'-O— oder 5'-O—Blockierungsgruppe aus dem so erhaltenen Produkt;

(4) Kondensieren des entstandenen Produktes mit einem weiteren Nucleosid- oder Olignucleosid-phosphit der Formel (I), um ein Produkt der Formel (II) zu bilden, worin n eine ganze Zahl ist, die um einen Wert größer ist als die im Produkt nach Schritt (1);

(5) Oxydieren des entstandenen Phosphittriesters zu einem Phosphattriester;

(6) Entfernen der 3'-O— oder 5'-O—Blockierungsgruppe aus dem so erhaltenen Produkt; und

(7) nach Bedarf Wiederholen jedes der Schritte (4), (5) und (6) aufeinanderfolgend, um der Kette eine gewünschte Sequenz von Nucleosiden hinzuzufügen; mit der Maßgabe, daß Schritt (6) nach der Zugabe des letzten Nucleosides zur Kette weggelassen werden kann.

16. Verfahren nach Anspruch 15, worin die Phosphitverbindung der Formel (1) die Formel

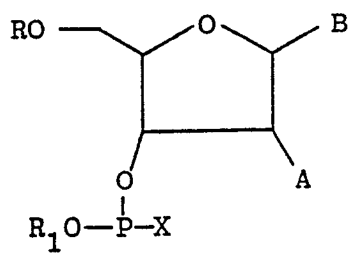

aufweist, worin R, $R_1$, X, A und B die in Anspruch 15 angeführte Bedeutung besitzen und worin die genannte Kondensation über das 5'OH des genannten Nucleosides oder Oligonucleotides erfolgt.

17. Verfahren nach Anspruch 15 oder 16, worin $R_1$ Niederalkyl ist.

18. Verfahren nach einem der Ansprüche 15 bis 17, worin das anorganische Polymer ein Silikagel ist.

19. Verfahren nach Anspruch 18, worin die freien Silanolhydroxygruppen am Silikagel vor der Kondensationsreaktion von Schritt (1) blockiert werden.

20. Verfahren nach Anspruch 18 oder 19, worin Ⓟ ein durch die Gruppe —$Z_1$NHCOZCO kovalent an das Nucleosid gebundenes Silika darstellt, worin Z und $Z_1$ jeweils Alkylen mit bis zu 5 C-Atomen bedeuten.

21. Verfahren nach einem Ansprüche 15 bis 20, worin A für H steht und R eine Tritylblockierungsgruppe bedeutet.

22. Verfahren nach Anspruch 21, worin die Tritylgruppe Dimethoxytrityl ist.

23. Verfahren nach einem der Ansprüche 15 bis 22, worin Schritt (2) und/oder (5) unter Verwendung von $I_2$ als Oxydationsmittel durchgeführt wird.

24. Verfahren nach einem der Ansprüche 15 bis 23, worin Schritt (3) und/oder Schritt (6) durch Reaktion mit einer Lewissäure durchgeführt, wird.

25. Verfahren nach Anspruch 24, worin die Lewissäure Zinkbromid ist.

26. Verfahren nach einem der Ansprüche 15 bis 25, umfassend den weiteren Schritt (8) des Hydrolysierens des Phosphattriesterproduktes, um das Oligonucleotid oder Polynucleotid und das anorganische Polymer zu bilden und das Oligonucleotid oder Polynucleotid daraus zu entfernen.

27. Verwendung einer Verbindung der Formel

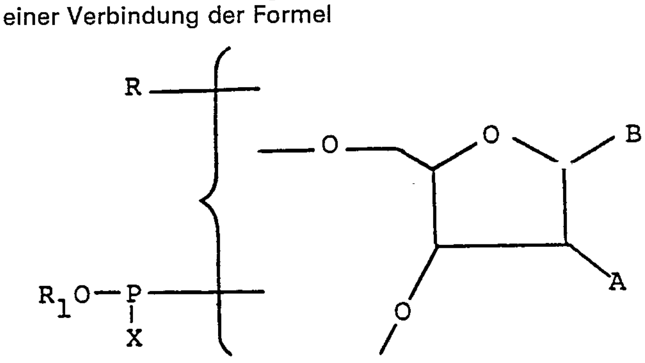

worin B eine Nucleosid- oder Desoxynucleosidbase bedeutet; R H oder eine Blockierungsgruppe ist; A für H oder OR steht; $R_1$ einen Hydrocarbylrest mit bis zu 10 C-Atomen darstellt; und X eine sekundäre Aminogruppe ist, in einem Verfahren zur Synthese eines längerkettigen Oligonucleotides oder Polynucleotides.

28. Die Erfindung nach Anspruch 27, worin die genannte Verbindung die Formel

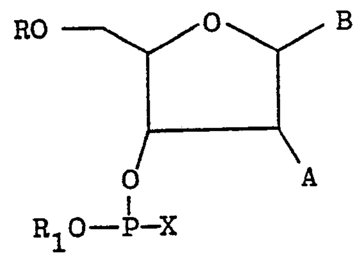

aufweist, worin B eine Nucleosid- oder Desoxynucleosidbase ist; R H oder eine Blockierungsgruppe darstellt; A für H oder OR steht; $R_1$ Niederalkyl ist; und X eine sekundäre Aminogruppe bedeutet, die durch Entfernung des H-Atoms des sekundären Aminostickstoffs einer heterocyclischen Stickstoffverbindung gebildet ist.

29. Die Erfindung nach Anspruch 28, worin die sekundäre Aminogruppe Tetrazolyl, Nitroimidazolyl oder Triazolyl bedeutet.

30. Die Erfindung nach einem der Ansprüche 27 bis 29, worin B für Thymin, Cytosin, Adenin oder Guanin steht.

31. Die Erfindung nach einem der Ansprüche 27 bis 30, worin R eine Tritylgruppe darstellt.

32. Die Erfindung nach einem der Ansprüche 27 bis 30, worin R Di-p-Anisylphenylmethyl oder p-Anisylphenylmethyl ist.

33. Die Erfindung nach einem der Ansprüche 27 bis 32, worin A für H steht.

**Revendications pour les Etats contractants: BE CH DE FR GB IT LI NL**

1. Procédé pour préparer un polymère minéral modifié, représenté par la formule:

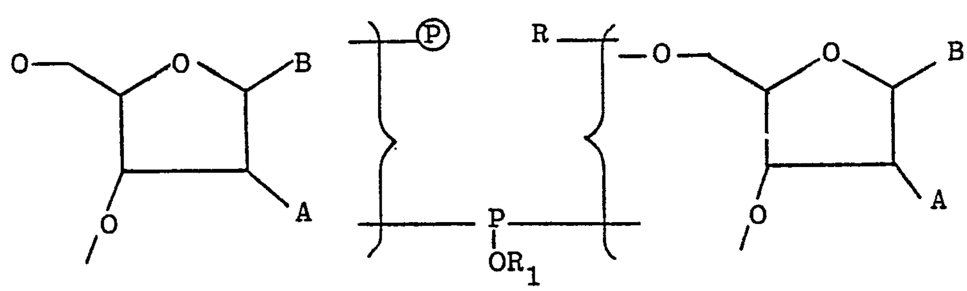

dans laquelle Ⓟ représente un polymère minéral relié à la position 3'-O ou 5'-O du nucléoside par un liaison de covalence hydrolysable; R représente H ou un groupe de blocage; $R_1$ est un radical hydrocarbyle contenant jusqu'à 10 atomes de carbone; chaque B représente une base de type nucléoside ou désoxynucléoside; et chaque symbole A représente H ou OR;
ce procédé comprenant la condensation d'un composé de formule:

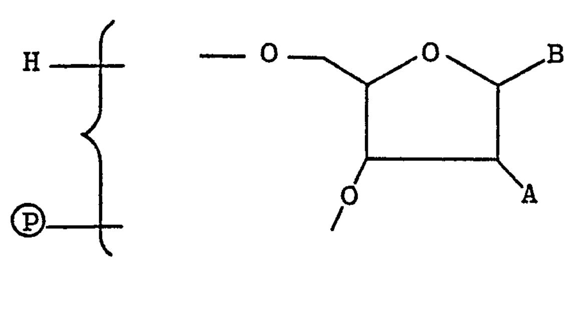

avec un composé de formule:

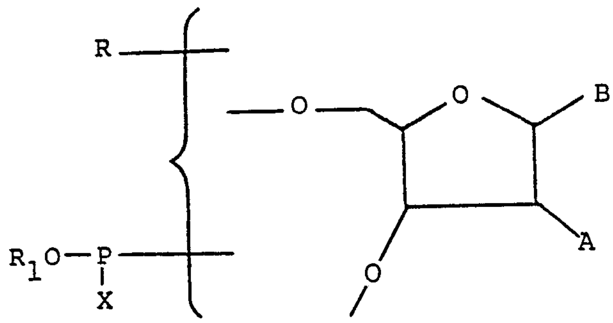

où A, B, Ⓟ, R et $R_1$ sont comme précédemment définis, et X représente un groupe amino secondaire fixé par l'intermédiaire de l'azote du groupe amino.

2. Procédé selon la revendication 1, dans lequel on condense un composé de formule:

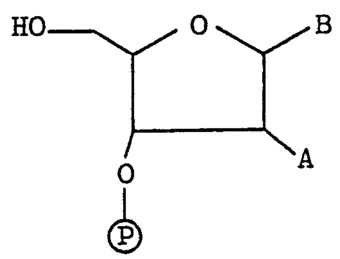

avec un composé de formule:

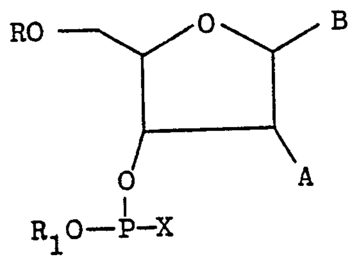

où A, B, Ⓟ, X, R et $R_1$ sont comme définis à la revendication 1, pour préparer un polymère minéral modifié de formule:

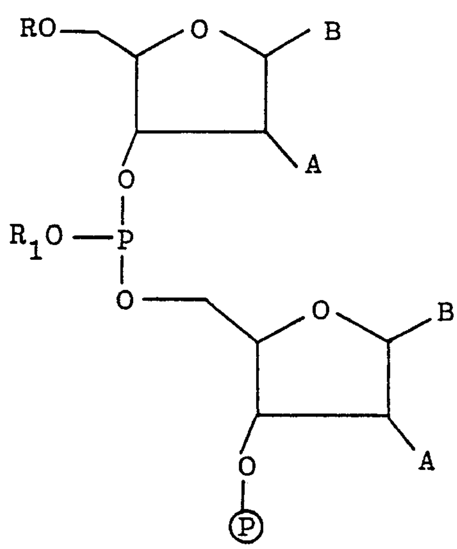

3. Procédé selon la revendication 1 ou la revendication 2, dans lequel $R_1$ représente un groupe alkyle inférieur.

4. Procédé selon l'une quelconque des revendications précédentes, dans lequel A représente H.

5. Procédé selon l'une quelconque des revendications précédentes, dans lequel B est choisi parmi l'adénine, la guanine, la cytosine, l'uracile et la thymine.

6. Procédé selon l'une quelconque des revendications précédentes, dans lequel Ⓟ est un gel de silice.

7. Procédé selon la revendication 6, dans lequel les groupes hydroxyles de silanol fixés sur le gel de silice sont protégés par un groupe de blocage.

8. Procédé selon la revendication 6 ou la revendication 7, dans lequel la silice est macroporeuse det a des particules dont la dimension se situe entre 5 et 1000 µm.

9. Procédé selon l'une quelconque des revendications précédentes, dans lequel X représente un groupe amino secondaire qui est formé par l'enlèvement de l'atome d'hydrogène d'un azote cyclique d'un composé hétérocyclique azoté, qui contient des liaisons insaturées dans la structure cyclique.

10. Procédé selon la revendication 9, dans lequel ledit composé hétérocyclique azoté est choisi parmi un tétrazole, un triazole et du nitroimidazole.

11. Procédé selon l'une quelconque des revendications précédentes, dans lequel le groupe de protection par blocage fixé sur 5'-O est un groupe trityle.

12. Procédé selon la revendication 11, dans lequel le groupe trityle est un groupe diméthoxytrityle ou monométhoxytrityle.

13. Procédé selon la revendication 6, ou selon l'une quelconque des revendications 7 à 12 quand elle dépend de la revendication 6, dans lequel Ⓟ est relié à la position 3'-O ou 5'-O du nucléoside par une liaison de covalence, hydrolysable à l'aide d'une base, de formule:

$$-(CH_2)_nNHCO-Z-CO-$$

dans laquelle n est un nombre entier valant 1 à 5 et Z représente un radical hydrocarbyle divalent.

14. Procédé pour préparer un polymère minéral modifié, de formule

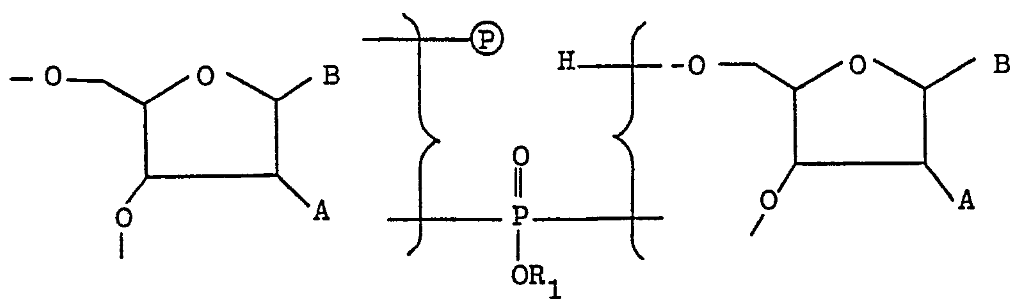

dans laquelle Ⓟ, A, B, R et $R_1$ sont comme définis dans la revendication 1, ce procédé comprenant le préparation d'un polymère minéral modifié, obtenu par le procédé selon l'une quelconque des revendications précédentes, puis l'oxydation du chaînon, phosphite en un chaînon phosphate, et l'enlèvement du groupe protecteur de blocage, fixé en 3'-O ou en 5'-O, du produit ainsi obtenu.

15. Composé de formule:

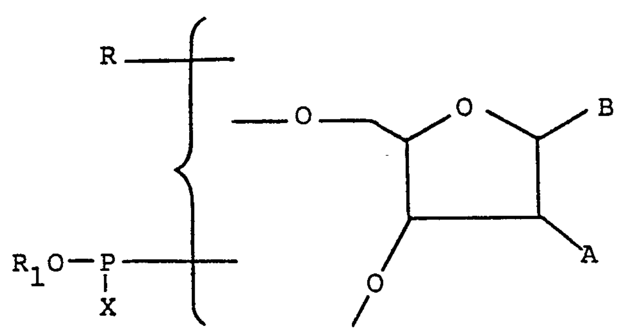

dans laquelle B représente un basé de type nucléoside ou désoxynucléoside; R représente H ou un groupe de protection par blocage, A représente H ou OR; $R_1$ représente un radical hydrocarbyle contenant jusqu'à 10 atomes de carbone; et X est un groupe amino secondaire.

16. Composé de formule

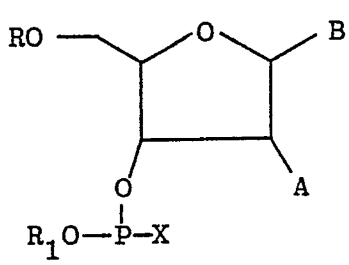

dans laquelle B représente un base de type nucléoside ou désoxynucléoside; R représente H ou un groupe de protection par blocage; A représente H ou OR; $R_1$ est un groupe alkyle inférieur; et X est un groupe amino secondaire, formé par enlèvement de l'atome de H de l'azote de groupe amino secondaire d'un composé hétérocyclic azoté.

17. Composé selon la revendication 16, dans lequel le groupe amino secondaire est un groupe tétrazolyle, nitroimidazolyle ou triazolyle.

18. Composé selon l'une quelconque des revendications 16 ou 17, dans lequel B représente la thymine, la cytosine, l'adénine ou la guanine.

19. Composé selon l'une quelconque des revendications 16 à 18, dans lequel R est un groupe trityle.

20. Composé selon l'une quelconque des revendications 16 à 18, dans lequel R représente un groupe di-p-anisylphénylméthyle ou p-anisylphénylméthyle.

21. Procédé pour la production d'un oligonucléotide ou d'un polynucléotide, ce procédé comprenant les étapes consistant à:

(1) condenser le groupe OH fixé en position 3' ou 5' d'un nucléoside ou d'un oligonucléotide relié par covalence à un polymère minéral à l'aide d'un agent de couplage sur les positions 5'-O ou 3'-O, respectivement, dudit nucléoside ou dudit nucléolite avec un phosphite de formule (I)

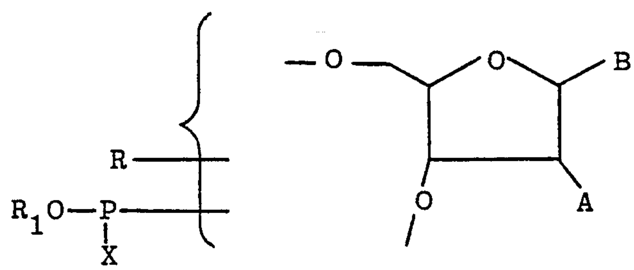

(I)

dans laquelle R représente un groupe de protection par blocage; B représente un base de type nucléoside ou désoxynucléoside; A représente H ou OR; $R_1$ est un radical hydrocarbyle contenant jusqu'à 10 atomes de carbone; et X représente un groupé amino secondaire; pour former un produit de formule (II)

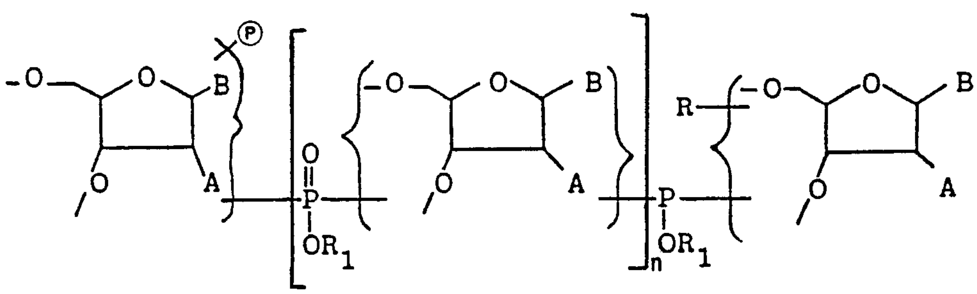

(II)

dans laquelle Ⓟ représente le polymère minéral, et n est un nombre entier valant au moins 1;

(2) oxyder le chaînon phosphite terminal du produit de l'étape (1) en un chaînon phosphate;

(3) enlever le groupe de protection par blocage de la position 3'-O our 5'-O du produit ainsi obtenu;

(4) condenser le produit résultant avec un autre phosphite de nucléoside ou d'oligonucléoside de formule (I), pour former un produit de formule (II) dans laquelle n est un nombre entier dont la valeur est supérieure d'une unité à la valeur de n dans le produit de l'étape (1);

(5) oxyder le triester phosphitique résultant en un triester de type phosphate;

(6) enlever du produit ainsi obtenu le groupe de protection par blocage de la position 3'-O our 5'-O; et

(7) si on le désire, répéter chacune des étapes (4), (5) et (6) successivement pour ajouter une séquence voulue des nucléosides à la chaîne;

à la condition de pouvoir omettre l'étape (6) après l'addition du nucléoside final sur la chaîne.

22. Procédé selon la revendication 21, dans lequel les phosphite de formule (I) a pour formule

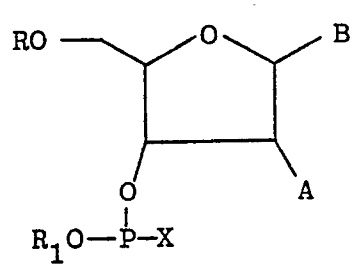

dans laquelle R, $R_1$, X, A et B sont tels que définis à la revendication 15, et dans lequel ladite condensation se produit par l'intermédiaire du groupe 5'-OH dudit nucléoside ou oligonucléotide.

23. Procédé selon la revendication 21 ou la revendication 22, dans lequel $R_1$ représente un groupe alkyle inférieur.

24. Procédé selon l'une quelconque des revendications 21 à 23, dans lequel le polymnère minéral est un gel de silice.

25. Procédé selon la revendication 24, dans lequel les groupes hydroxyles libres de silanol fixés sur le gel de silice sont protégés par blocage avant la réaction de condensation de l'étape (1).

26. Procédé selon la revendication 24 ou la revendication 25, dans lequel Ⓟ est de la silice liée par covalence au nucléoside par l'intermédiaire du groupe — —$Z_1NHCOZCO$—, dans lequel Z et $Z_1$ sont chacun un groupe alkylène ayant jusqu'à 5 atomes de carbone.

27. Procédé selon l'une quelconque des revendications 21 à 26, dans lequel A représente H et R représente un groupe trityle de protection par blocage.

28. Procédé selon la revendication 27, dans lequel le groupe trityle est le groupe diméthoxytrityle.

29. Procédé selon l'une quelconque des revendications 21 à 28, dans lequel on conduit l'étape (2) et/ou (5) en utilisant $I_2$ comme agent oxydant.

30. Procédé selon l'une quelconque des revendications 21 à 29, dans lequel on conduit l'étape (3) et/ou (6) par la réaction avec un acide de Lewis.

31. Procédé selon la revendication 30, dans lequel l'acide de Lewis est du bromure de zinc.

32. Procédé selon l'une quelconque des revendications 21 à 31, comprenant l'étape supplémentaire (8), consistant à hydrolyser le triester phosphorique produit, pou former l'oligonucléotide ou le polynucléotide et le polymére minéral, et à en séparer l'oligonucléotide ou le polynucléotide.

**Revendications pour l'Etat contractant: AT**

1. Procédé pour préparer un polymère minéral modifié, représenté par la formule:

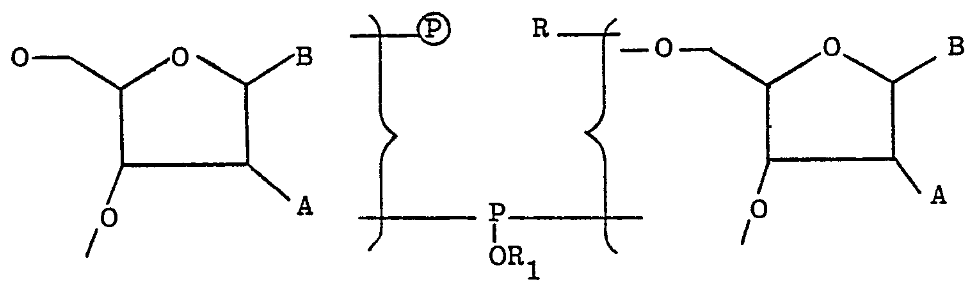

dans laquelle Ⓟ représente un polymère minéral relié à la position 3'-O ou 5'-O du nucléoside par un liaison de covalence hydrolysable; R représente H ou un groupe de blocage; $R_1$ est un radical hydrocarbyle contenant jusqu'à 10 atomes de carbone; chaque B représente une base de type nucléoside ou désoxynucléoside; et chaque symbole A représente H ou OR;
ce procédé comprenant la condensation d'un composé de formule:

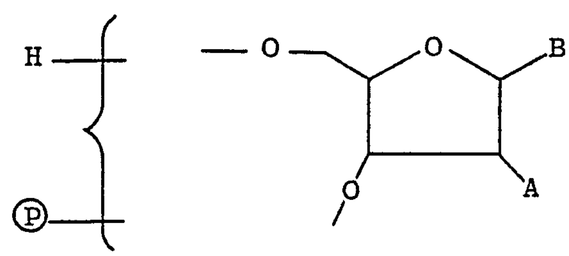

avec un composé de formule:

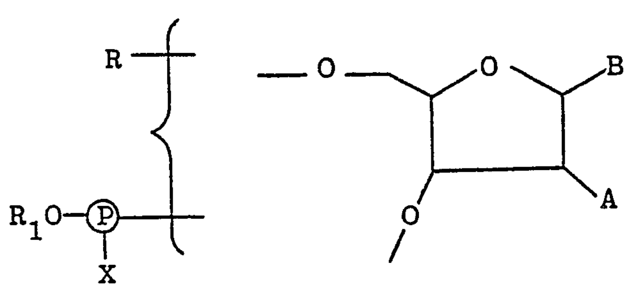

où A, B, Ⓟ, R et $R_1$ sont comme précédemment définis, et X représente un groupe amino secondaire fixé par l'intermédiaire de l'azote du groupe amino.

2. Procédé selon la revendication 1, dans lequel on condense un composé de formule:

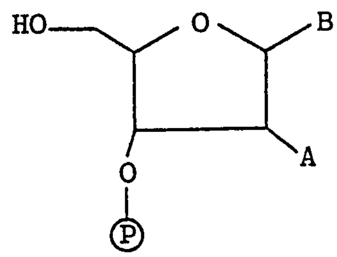

avec un composé de formule:

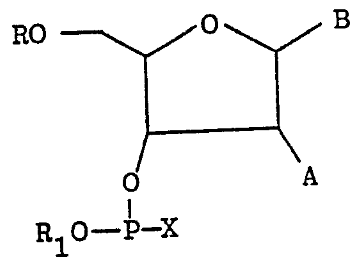

où A, B, Ⓟ, X, R et $R_1$ sont comme définis à la revendication 1, pour préparer un polymère minéral modifié de formule:

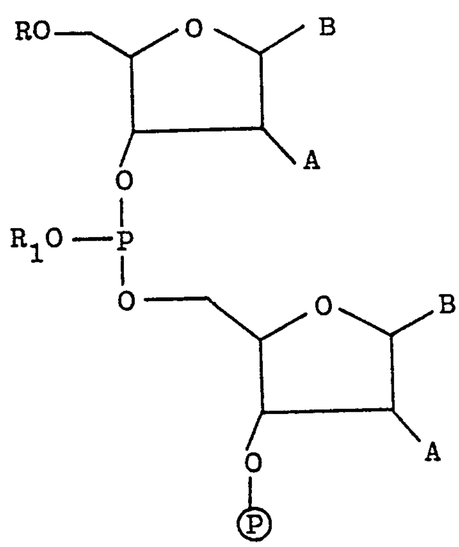

3. Procédé selon la revendication 1 ou la revendication 3, dans lequel $R_1$ représente un groupe alkyle inférieur.

4. Procédé selon l'une quelconque des revendications précédentes, dans lequel A représente H.

5. Procédé selon l'une quelconque des revendications précédentes, dans lequel B est choisi parmi l'adénine, la guanine, la cytosine, l'uracile et la thymine.

6. Procédé selon l'une quelconque des revendications précédentes, dans lequel P est un gel de silice.

7. Procédé selon la revendication 6, dans lequel les groupes hydroxyles de silanol fixés sur le gel de silice sont protégés par un groupe de blocage.

8. Procédé selon la revendication 6 ou la revendication 7, dans lequel la silice est macroporeuse det a des particules dont la dimension se situe entre 5 et 1000 µm.

9. Procédé selon l'une quelconque des revendications précédentes, dans lequel X représente un groupe amino secondaire qui est formé par l'enlèvement de l'atome d'hydrogène d'un azote cyclique d'un composé hétérocyclique azoté, qui contient des liaisons insaturées dans la structure cyclique.

10. Procédé selon la revendication 9, dans lequel ledit composé hétérocyclique azoté est choisi parmi un tétrazole, un triazole et du nitroimidazole.

11. Procédé selon l'une quelconque des revendications précédentes, dans lequel le groupe de protection par blocage fixé sur 5'-O est un groupe trityle.

12. Procédé selon la revendication 11, dans lequel le groupe trityle est un groupe diméthoxytrityle ou monométhoxytrityle.

13. Procédé selon la revendication 6, ou selon l'une quelconque des revendications 7 à 12 quand elle dépend de la revendication 6, dans lequel Ⓟ est relié à la position 3'-O ou 5'-O du nucléoside par une liaison de covlance, hydrolysable à l'aide d'une base, de formule:

$$—(CH_2)_nNHCO—Z—CO—$$

dans laquelle n est un nombre entier valant 1 à 5 et Z représente un radical hydrocarbyle divalent.

14. Procédé pour préparer un polymère minéral modifié, de formule

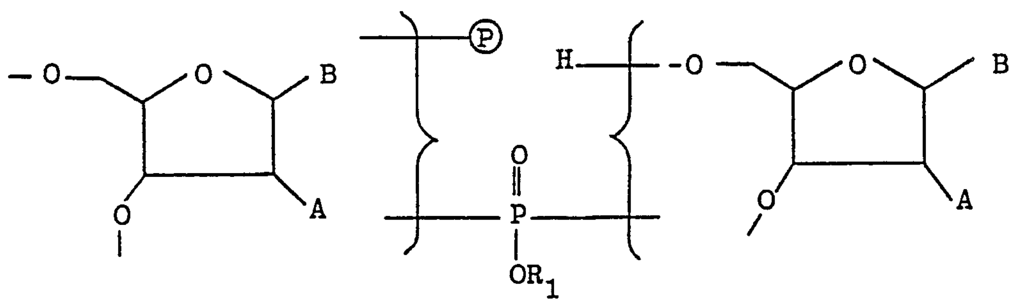

dans laquelle Ⓟ, A, B, R et $R_1$ sont comme définis dans la revendication 1, ce procédé comprenant le préparation d'un polymère minéral modifié, obtenu par le procédé selon l'une quelconques des revendications précédentes, puis l'oxydation du chaînon, phosphite en un chaînon phosphate, et l'enlèvement du groupe protecteur de blocage, fixé en 3'-O ou en 5'-O, du produit ainsi obtenu.

15. Procédé pour la production d'un oligonucléotide ou d'un polynucléotide, ce procédé comprenant les étapes consistant à:

(1) condenser le groupe 3'-OH ou 5'-OH d'un nucléoside ou d'un oligonucléotide relié par covalence à un polymère minéral à l'aide d'un agent de couplage sur les positions 5'-O ou 3'-O, respectivement, dudit nucléoside ou oligonucléotide avec un phosphite de formule (I)

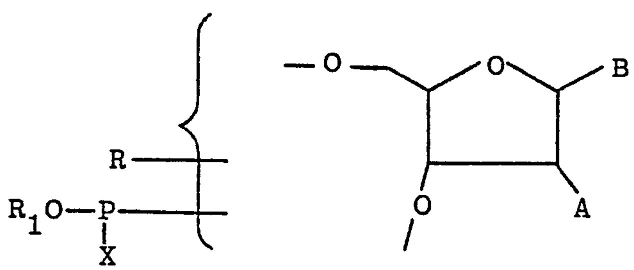

(I)

dans laquelle R représente un groupe de protection par blocage; B représente une base de type nucléoside ou désoxynucléoside; A représente H ou OR; $R_1$ est un radical hydrocarbyle contenant jusqu'à 10 atomes de carbone; et X représente un groupé amino secondaire; pour former un produit de formule (II)

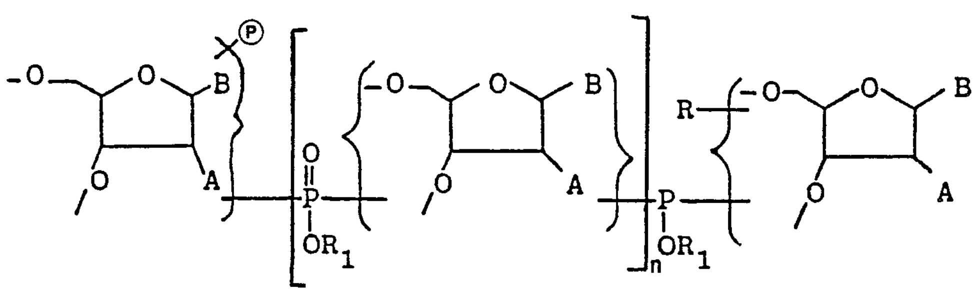

(II)

dans laquelle Ⓟ représente le polymère minéral, et n est un nombre entier valant au moins 1;

(2) oxyder le chaînon phosphite terminal du produit de l'étape (1) en un chaînon phosphate;

(3) enlever le groupe de protection par blocage de la position 3'-O ou 5'-O du produit ainsi obtenu;

(4) condenser le produit résultant avec un autre phosphite de nucléoside ou d'oligonucléoside de formule (I), pour former un produit de formule (II) dans laquelle n est un nombre entier dont la valeur est supérieure d'une unité à la valeur de n dans le produit de l'étape (1);

(5) oxyder le triester phosphitique résultant en un triester de type phosphate;

(6) enlever du produit ainsi obtenu le groupe de protection par blocage de la position 3'-O our 5'-O; et

(7) si on le désire, répéter chacune des étapes (4), (5) et (6) successivement pour ajouter une séquence voulue des nucléosides à la chaîne;

à la condition qu'on puisse omettre les étapes (5) et (6) après l'addition du nucléoside final sur la chaîne;

16. Procédé selon la revendication 15, dans lequel les phosphite de formule (I) a pour formule

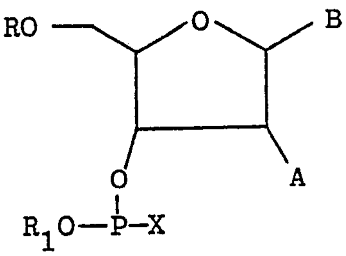

dans laquelle R, $R_1$, X, A et B sont tels que définis à la revendication 15, et dans lequel ladite condensation se produit par l'intermédiaire du groupe 5'-OH dudit nucléoside ou oligonucléotide.

17. Procédé selon la revendication 15 ou la revendication 16, dans lequel $R_1$ représente un groupe alkyle inférieur.

18. Procédé selon l'une quelconque des revendications 15 à 17, dans lequel le polymère minéral est un gel de silice.

19. Procédé selon la revendication 18, dans lequel les groupes hydroxyles libres de silanol fixés sur le gel de silice sont protégés par blocage avant la réaction de condensation de l'étape (1).

20. Procédé selon la revendication 18 ou la revendication 19, dans lequel Ⓟ est de la silice liée par covalence au nucléoside par l'intermédiaire du groupe $—Z_1NHCOZCO—$, dans lequel Z et $Z_1$ sont chacun un groupe alkylène ayant jusqu'à 5 atomes de carbone.

21. Procédé selon l'une quelconque des revendications 15 à 20, dans lequel A représente H et R représente un groupe trityle de protection par blocage.

22. Procédé selon la revendication 21, dans lequel le groupe trityle est le groupe diméthoxytrityle.

23. Procédé selon l'une quelconque des revendications 15 à 22, dans lequel on conduit l'étape (2) et/ou (5) en utilisant $I_2$ comme agent oxydant.

24. Procédé selon l'une quelconque des revendications 15 à 23, dans lequel on conduit l'étape (3) et/ou (6) par la réaction avec un acide de Lewis.

25. Procédé selon la revendication 24, dans lequel l'acide de Lewis est du bromure de zinc.

26. Procédé selon l'une quelconque des revendications 15 à 25, comprenant l'étape supplémentaire (8), consistant à hydrolyser le triester de type phosphate produit, pour former l'oligonucléotide ou le polynucléotide et le polymére organique, et à en séparer l'oligonucléotide ou le polynucléotide.

27. Utilisation, dans un procédé de synthèse d'un oligonucléotide ou polynucléotide à plus longue chaîne, d'un composé de formule

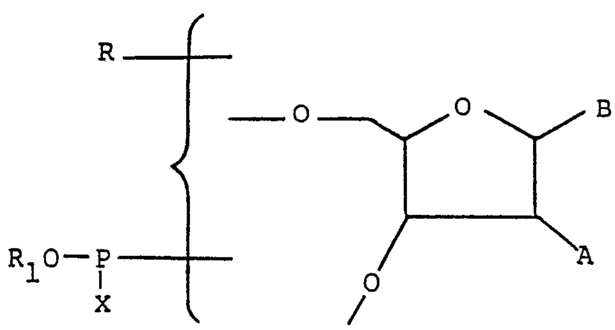

dans laquelle B représente une base de type nucléoside ou désoxynucléoside; R représente H ou un groupe de protection par blocage, A représente H ou OR; $R_1$ représente un radical hydrocarbyle contenant jusqu'à 10 atomes de carbone; et X est un groupe amino secondaire.

28. Utilisation selon la revendication 27, dans laquelle ledit composé répond à la formule

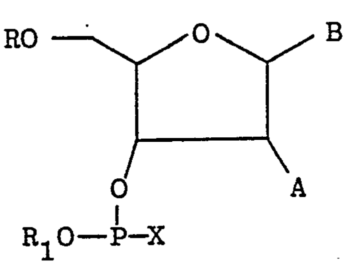

dans laquelle B est une base de type nucléoside ou désoxynucléoside; R représente H ou un groupe protecteur par blocage, A représente H ou OR; $R_1$ représente un groupe alkyle inférieur; et X est un groupe amino secondaire, formé par enlèvement de l'atome de H d'un azote de groupe amino secondaire d'un composé hétérocyclique azoté.

29. Utilisation selon la revendication 28, dans laquelle le groupe amino secondaire est un groupe têtrazolyle, nitroimidazolyle ou triazolyle.

30. Utilisation selon l'une quelconque des revendications 27 à 29, dans laquelle B représente la thymine, la cytosine, l'adénine ou la guanine.

31. Utilisation selon l'une quelconque des revendications 27 à 30, dans laquelle R représente un groupe trityle.

32. Utilisation selon l'une quelconque des revendications 27 à 30, dans laquelle R représente un groupe di-p-anisylphénylméthyle ou p-ainsylphénylméthyl.

33. Utilisation selon l'une quelconque des revendications 27 à 32, dans laquelle A représente H.

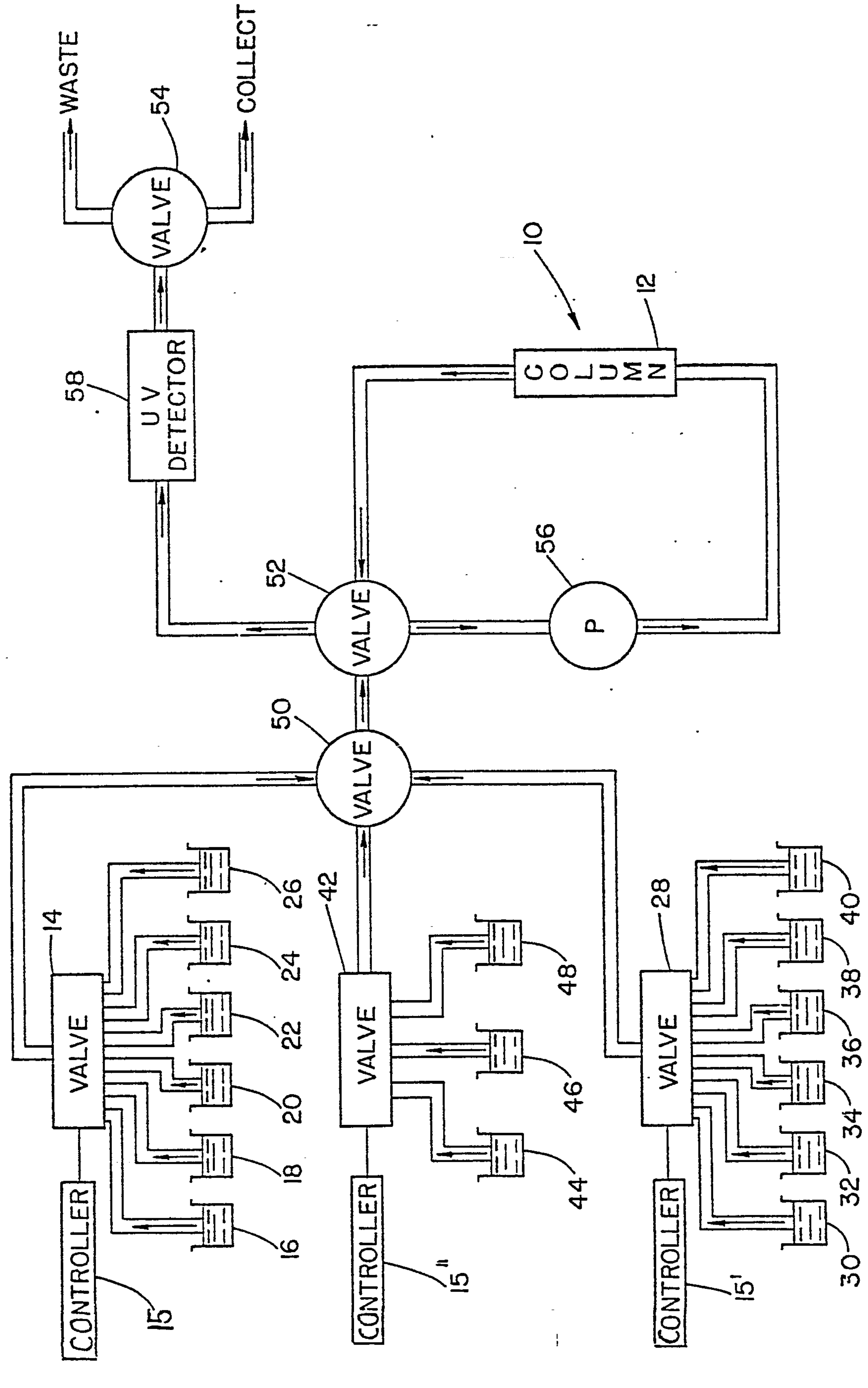
WASTE
COLLECT
54
VALVE
58
U V DETECTOR
10
12
COLUMN
52
VALVE
56
P
50
VALVE
14
VALVE
42
VALVE
28
VALVE
26
24
22
20
18
16
48
46
44
40
38
36
34
32
30
CONTROLLER
15
CONTROLLER
15''
CONTROLLER
15'